(19) Europäisches Patentamt
European Patent Office
Office européen des brevets

(11) Publication number: 0 156 233
A2

# (12) EUROPEAN PATENT APPLICATION

(21) Application number: 85102787.0

(22) Date of filing: 12.03.85

(51) Int. Cl.⁴: A 61 K 31/35

(30) Priority: 19.03.84 US 590815
21.12.84 US 685102

(43) Date of publication of application:
02.10.85 Bulletin 85/40

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Goldenberg, Marvin M.
721 Shackamaxon Drive
Westfield, N.J. 07090(US)

(74) Representative: Blum, Rudolf Emil Ernst et al,
c/o E. Blum & Co Patentanwälte Vorderberg 11
CH-8044 Zürich(CH)

## (54) Use of leukotriene antagonists for producing cytoprotective pharmaceutical compositions and process for producing cytoprotective pharmaceutical compositions.

(57) Leukotriene antagonists have been found to be useful for inducing cytoprotection via an unspecified mechanism. Specific therapeutic utilities include, erosive gastritis, erosive esophagitis, inflammatory bowel disease, and induced hemorrhagic gastric erosions such as those caused by indomethacin or ethanol.

The leukotriene antagonists are used to produce cytoprotective pharmaceutical compositions. In a process for producing cytoprotective pharmaceutical compositions the leukotriene antagonists are incorporated into a corresponding composition in a cytoprotective effective amount.

EP 0 156 233 A2

TITLE OF THE INVENTION

Use of LEUKOTRIENE ANTAGONISTS for producing CYTOPROTECTIVE pharmaceutical compositions and process for producing CYTOPROTECTIVE pharmaceutical compositions.

CROSS-REFERENCE

This is a continuation-in-part of copending application U.S.S.N. 590,815, filed March 19, 1984.

Compounds used herein are disclosed in copending application U.S.S.N. 520,052, filed August 5, 1983 and in U.S. 3,882,148 which are incorporated herein by reference.

BACKGROUND OF THE INVENTION

The present invention concerns the use of leukotriene antagonists in a process for the production of cytoprotective pharmaceutical compositions.

Furthermore, the present invention concerns a process for the production of a cytoprotective pharmaceutical composition which contains as active ingredient a cytoprotective effective amount of at least one leukotriene antagonist.

The pharmaceutical composition prepared according to the inventive use of leukotriene antagonists, respectively prepared according to the inventive process can be administered in a mammal, especially in man, for inducing cytoprotection. The used leukotriene antagonists are useful as anti-asthmatic, anti-allergic, and anti-inflammatory agents and are useful in treating allergic rhinitis and chronic bronchitis and for amelioration of skin diseases like psoriasis and atopic eczema. These compounds are also useful to antagonize or inhibit the properties of leukotrienes relating to cardiovascular and vascular systems.

Prior to this invention, there was no evidence in the literature that the leukotriene antagonists were antiulcerogenic/cytoprotective.

SUMMARY OF THE INVENTION

The present invention concerns the use of leukotriene antagonists for the production of cytoprotective pharmaceutical compositions. Furthermore, the present invention concerns a process for the preparation of a cytroprotective pharmaceutical composition which contains as active ingredient a cytoprotective effective amount of a leukotriene antagonist.

Preferred leukotriene antagonists which are used according to the present invention, respectively the cytoprotective effective component used in the inventive process, are compounds having the formulae XI, XII or XIII

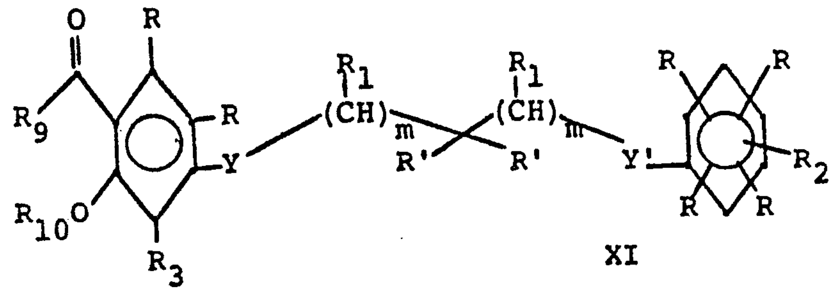

XI

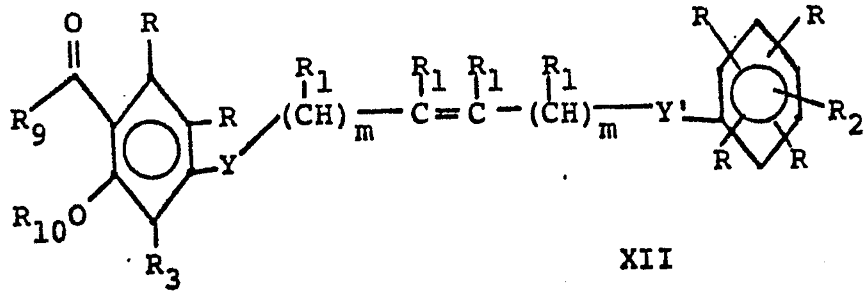

XII

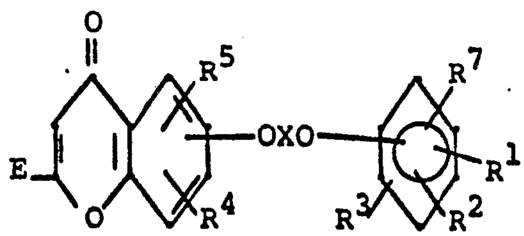

XIII

or are the compounds described in the USA patent 3,882,148 as well as certain propionic acid analogs of the compound described therein or are pharmaceutically acceptable salts of the above stated classes of compounds.

DETAILED DESCRIPTION OF THE INVENTION

This invention is directed to the use of leukotriene antagonists generally, and specifically compounds of formulae XI and XII and those of U.S.

3,882,148 as cytoprotective agents. Because of their cytoprotective nature, these compounds may be used to treat or prevent disease states such as erosive gastritis; erosive esophagitis; inflammatory bowel disease; ethanol-induced hemorrhagic erosions; hepatic ischemia; noxious agent induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile salt induced pancreatic or gastric damage; trauma- or stress-induced cell damage; and glycerol-induced renal failure.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that

cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay.

A. Ethanol-Induced Lesion Assay

Twenty-four hour fasted Sprague-Dawley (S.D.) rats are perorally (p.o.) dosed with 1.0 ml absolute ethanol. Fifteen min. prior to ethanol administration, groups of rats each receive either an aqueous vehicle (aqueous methylcellulose 5% wt.) or the test compound at various doses perorally. One hour later, the animals are sacrificed and stomach mucosae are examined for resulting lesions.

B. Indomethacin-Induced Ulcer Assay

Indomethacin, 10 mg/kg p.o., is used to induce ulcers in 24 hr. fasted S.D. rats. Fifteen minutes prior to indomethacin administration, groups of rats each receive either an aqueous vehicle (5% wt. methylcellulose) or the test compound at various doses perorally. Four hours later the animals are sacrificed and stomach mucosae are examined for resulting ulcers.

As cytoprotective agents, the leukotriene antagonists may general · be administered at a dosage range of 0.001 mg/kg to 100 mg/kg of body weight. The exact amount of inhibitor to be used will depend

on, inter alia, whether it is being administered to heal damaged cells or to avoid future damage, on the nature of the damaged cells (e.g., gastro-intestinal ulcerations vs. nephrotic necrosis), and on the nature of the causative agent.

The effective daily dosage level for compounds of Formulae XI or XII inducing cytoprotection in mammals, especially humans, will range from about 0.001 mg/kg to about 100 mg/kg, preferably from about 0.01 mg/kg to about 30 mg/kg. The dosage may be administered in single or divided individual doses.

Any suitable route of administration may be employed for providing a mammal, especially a human, with a cytoprotectively effective dosage of a leukotriene antagonist. For example, oral, rectal, transdermal, parenteral, intramuscular, intraveneous and the like may be employed. Dosage forms include tablets, troches, dispersions, suspensions, solutions, capsules and the like.

In practical use, leukotriene antagonists can be combined as the active ingredient in intimate admixture with a pharmaceutical carrier according to conventional pharmaceutical compounding techniques. The carrier may take a wide variety of forms depending on the form of preparation desired for administration, e.g., oral or intravenous. In preparing the compositions for oral dosage form, any of the usual pharmaceutical media may be employed, such as, for example, water glycols, oils, alcohols, flavoring agents, preservatives, coloring agents and the like in the case of oral liquid preparations,

such as, for example, suspensions, elixirs and solutions; or carriers such as starches, sugars, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the leukotriene antagonists may also be administered by controlled release means and/or delivery devices such as those described in U.S. Patent Nos. 3,845,770; 3,916,899; 3,536,809; 3,598,123; 3,630,200 and 4,008,719.

In preparing suitable dosage forms, conventional compounding procedures and ingredients e.g. diluents, carriers, etc. may be used. The following are examples of representative pharmaceutical dosage forms for leukotriene antagonists:

| Injectable Suspension | mg/ml |
|---|---|
| Leukotriene antagonist | 0.07-70 |
| Methylcellulose | 5.0 |
| Tween 80 | 0.5 |
| Benzyl alcohol | 9.0 |
| Methyl paraben | 1.8 |
| Propyl paraben | 0.2 |

Water for injection to a total volume of 10 ml

| Tablet | mg/tablet |
|---|---|
| Leukotriene antagonist | 0.07-70 |
| Microcrystalline Cellulose | 0-349.8 |
| Providone | 14.0 |
| Microcrystalline Cellulose | 90.0 |
| Pregelatinized Starch | 43.5 |
| Magnesium Stearate | 2-2.5 |
| | 500 |

| Capsule | mg/capsule |
|---|---|
| Leukotriene antagonist | 0.07-70 |
| Lactose Powder | 248.5-598.3 |
| Magnesium Stearate | 1-1.5 |
| | 600 |

In addition to the leukotriene antagonists, the pharmaceutical compositions can also contain other active ingredients, such as cyclooxygenase inhibitors or non-steroidal anti-inflammatory drugs (NSAIDs). NSAIDs can be characterized into five groups:

(1) the propionic acid derivatives;

(2) the acetic acid derivatives;

(3) the fenamic acid derivatives;

(4) the biphenylcarboxylic acid derivatives; and

(5) the oxicams

or a pharmaceutically acceptable salt thereof.

The propionic acid derivatives which may be used comprise: ibuprofen, ibuprufen aluminum, indoprufen, ketoprufen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having similar analgesic and anti-inflammatory properties are also intended to be included in this group.

Thus, "propionic acid derivatives" as defined herein are non-narcotic analgesics/non-steroidal anti-inflammatory drugs having a free $-CH(CH_3)COOH$ or $-CH_2CH_2COOH$ group (which optionally can be in the form of a pharmaceutically acceptable salt group, e.g., $-CH(CH_3)COO^-Na^+$ or $-CH_2CH_2COO^-Na^+$), typically attached directly or via a carbonyl function to a ring system, preferably to an aromatic ring system.

The acetic acid derivatives which may be used comprise: indomethacin, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structually related acetic acid derivatives having similar analgesic and anti-inflammatory properties are also intended to be encompassed by this group.

Thus, "acetic acid derivatives" as defined herein are non-narcotic analgesics/non-steroidal anti-inflammatory drugs having a free $-CH_2COOH$ group (which optionally can be in the form of a pharmaceutically acceptable salt group, e.g. $-CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-narcotic analgesics/non-steroidal anti-inflammatory drugs which contain the basic structure:

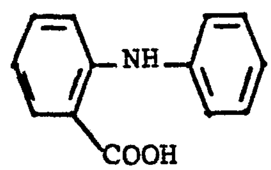

which can bear a variety of substituents and in which the free -COOH group can be in the form of a pharmaceutically acceptable salt group, e.g., $-COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

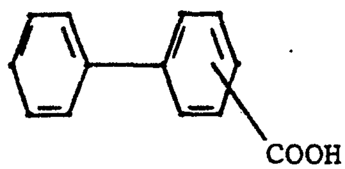

which can bear a variety of substituents and in which the free -COOH group can be in the form of a pharmaceutically acceptable salt group, e.g., $-COO^{-}Na^{+}$.

The oxicams which can be used in the present invention comprise: piroxicam, sudoxicam, isoxicam and 4-hydroxyl-1,2-benzothiazine 1,1-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

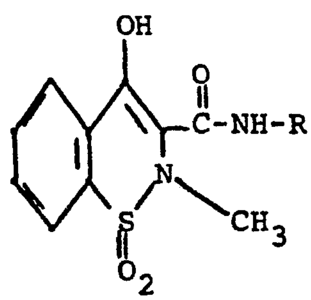

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs, designated by company code number, may also be used: 480156S, AA861, AD1491, AD1590, AFP802, AFP860, AHR6293, AI77B, AP504, AU8001, BAYo8276, BPPC, BW540C, BW755C, CHINOIN 127, CN100, CO893XX, CPP, D10242, DKA9, DV17, EB382, EGYT2829, EL508, F1044, FZ, GP53633, GP650, GV3658, HG/3, ITC1, ITF, ITF182, KB1043, KC8973, KCNTEI6090, KME4, LA2851, LT696, LU20884, M7074, MED15, MG18311, MR714, MR897, MY309, NO164, ONO3144, PR823, PV102, PV108, QZ16, R830, RS2131, RU16029, RU26559, RUB265, SCR152, SH440, SIR133, SIR136, SIR92, SPAS510, SQ27239, ST281, SX1032, SY6001, SaH46798, TA60, TAI901, TEI615, TVX2706, TVX960, TZI615, U60257, UR2310, WY23205, WY41770, YM09561, YM13162, YS1033, and ZK31945.

Finally, NSAIDs which may also be used include the salicylates, specifically aspirin, and the phenylbutazones and pharmaceutically acceptable salts thereof.

The leukotriene antagonists may also be used in combination with inhibitors of the biosynthesis of the leukotrienes such as those disclosed in copending application U.S. Serial No. 559,471, filed December 12, 1983 which is herein incorporated by reference and others known in the art. These pharmaceutical compositions may also contain as the second active ingredient, antihistaminic agents such as benadryl, dramamine, histadyl, phenergan and the like. Alternatively, they may include prostaglandin antagonists such as those disclosed in European Patent Application 11,067 or thromboxane antagonists such as those disclosed in U.S. 4,237,160. They may

also contain histidine decarboxylase inhibitors such as α-fluoromethylhistidine, described in U.S. 4,325,961. The leukotriene antagonists may also be advantageously combined with an $H_1$ or $H_2$-receptor antagonist, such as for instance cimetidine, ranitidine, terfenadine, famotidine, aminothiadiazoles disclosed in EP 81102976.8 and like compounds, such as those disclosed in U.S. Patent Nos. 4,283,408; 4,362,736; 4,394,508; European Patent Application No. 40,696 and a pending application, U.S.S.N. 301,616, filed September 14, 1981. Each of these references is herein incorporated by reference. The pharmaceutical compositions may also contain a $K^+/H^+$ ATPase inhibitor such as omeprazole, disclosed in U.S. Pat. 4,255,431, and the like. The weight ratio of the leukotriene antagonist to NSAID or other active ingredient may be varied and may range from 200:1 to about 1:200, an effective amount of each compound being used.

A further embodiment of this invention is a method of reducing the undesirable side effects of NSAIDs which comprises the concomitant administration of a cytoprotectively effective amount of a leukotriene antagonist, especially one of Formulae XI or XII or those of U.S. 3,882,148 (and certain propionic acid analogs thereof), and an NSAID. By concomitant is meant that the leukotriene antagonist is administered from 30 minutes prior up to 30 minutes after administration of the NSAID. Preferably, the leukotriene antagonist is administered prior to or simultaneously with the NSAID.

In the compositions and methods of this invention, the leukotriene antagonists particularly preferred are:

(ßS*, γR*)-sodium 4-(3-(4-acetyl-3-hydroxy-2-propylphenoxy)propylthio)-γ-hydroxy-ß-methyl-benzenebutanoate,

(ßS*, γR*) 4-(3-(4-acetyl-3-hydroxy-2-propylphenoxy)-propyloxy)-γ-hydroxy-ß-methyl-benzenebutanoic acid sodium salt,

(2,2-dimethyl-1-oxopropoxy)methyl 4-((3-(4-acetyl-3-hydroxy-2-propylphenoxy)propyl)sulfonyl)-γ-oxo-benzenebutanoate,

(ßS*, γR*)-sodium 4-((3-(4-acetyl-3-hydroxy-2-propylphenoxy)propyl)sulfonyl)-γ-hydroxy-ß-methylbenzenebutanoate,

4-((3-(4-acetyl-3-hydroxy-2-propylphenoxy)propyl)-sulfonyl)-γ-oxobenzenebutanoic acid,

4-((3-(4-acetyl-3-hydroxy-2-propylphenoxy)propyl)thio-γ-oxobenzenebutanenitrile,

7-[3-(4-acetyl-3-hydroxy-2-propylphenoxy)-2-hydroxy-propoxy]-4-oxo-8-propyl-4H-1-benzopyran-2-carboxylic acid,

7-[3-(4-acetyl-3-hydroxy-2-propylphenoxy)-2-hydroxyl-propyloxy]-4-oxo-8-propyl-4H-1-benzopyran-2-proprionic acid, and

7-[3-(4-acetyl-3-hydroxy-2-propylphenoxy)propyloxy]-4-oxo-8-propyl-4H-1-benzopyran-2-proprionic acid.

The latter three compounds are also known as FPL 55712, FPL 57231, and FPL 59257, respectively (Leukotrienes and Other Lipoxygenase Products, Samuelsson et al., ed., pp. 229-235, Raven Press, 1982).

Specific compounds useful in this invention are those of Formulae XI and XII:

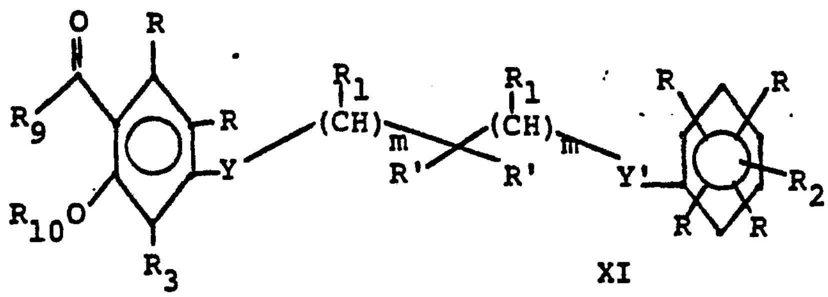

XI

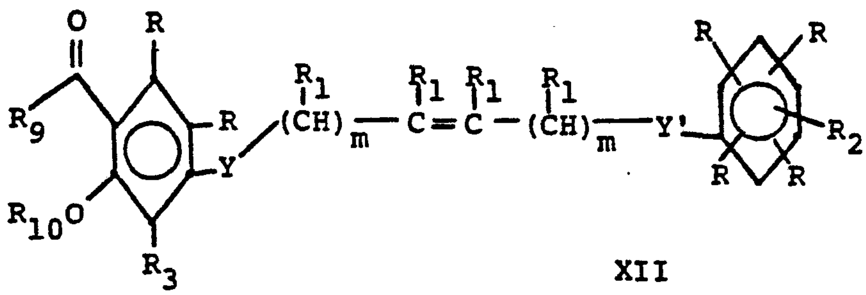

XII

wherein

each R is independently H, OH, alkyl of 1 to 6 carbon atoms which may be straight chain or branched; alkenyl of 2 to 6 carbon atoms which may be straight chain or branched; trifluoromethyl; alkoxy of 1 to 6

carbon atoms which may be straight chain or branched; SH; thioalkyl of 1 to 6 carbon atoms which may be straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R' is independently $R_4$; $OR_4$; $COOR_4$; $N(R_4)_2$; $SR_4$; $CH_2OR_4$; CHO; or together R' and R' are O; $CH_2$; or 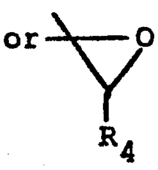 ;

Y is oxygen, sulfur, sulfoxide, sulfone; $\overset{O}{\overset{\|}{S}}=NR_{11}$ wherein $R_{11}$ is alkyl of 1-4 carbon atoms which may be straight chain or branched; $NR_{12}$ wherein $R_{12}$ is H, alkyl of 1-4 carbon atoms which may be straight chain or branched; or $N-\overset{O}{\overset{\|}{C}}-R_{13}$ wherein $R_{13}$ is alkyl of 1-4 carbon atoms which may be straight chain or branched, alkoxy of 1-4 carbon atoms which may be straight chain or branched; or N-CN;

Y' is Y, $-CH_2-$ or $-\overset{O}{\overset{\|}{C}}-$;

each $R_1$ is independently hydrogen or alkyl of 1-3 carbon atoms;

each m is independently an integer from 0-6; and

$R_2$ is 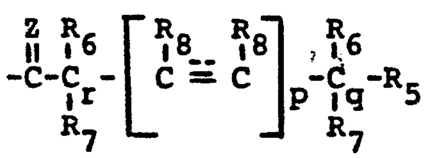

wherein Z is O, S, $CH_2$, H and OH, alkenyl of 1-4 carbons; or N-$R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of 1 to 3 carbon atoms, halogen, hydroxy, haloalkyl, COOH, CN, formyl or acyl of 1 to 6 carbon atoms;

each $R_6$ is independently H or alkyl of 1-4 carbons;

each $R_7$ is independently H, OH, or alkyl of 1-4 carbons;

each $R_8$ is independently H, or alkyl of 1-4 carbons, and is absent when a triple bond is present;

$R_5$ is $COOR_4$; $CH_2OH$; CHO; tetrazole; $NHSO_2R_{14}$; $CONHSO_2R_{14}$; hydroxymethylketone; CN; $CON(R_7)_2$; a monocyclic or bicyclic heterocyclic ring containing an acidic hydroxyl group; or $COOR_{15}$ where $R_{15}$ is:

$$-(-CH_2)_s-\underset{R_6}{\overset{R_6}{\underset{|}{\overset{|}{C}}}}(CH_2)_s-R_{16}$$ wherein each s is

independently 0-3; $R_{16}$ is

A) a monocyclic or bicyclic heterocyclic radical containing from 3 to 12 nuclear carbon atoms and 1 or 2 nuclear heteroatoms selected from N and S with at least one being N, and with each ring in the heterocyclic radical being formed of 5 or 6 atoms, or

B) the radical W-$R_{17}$ wherein W is O, S or NH and $R_{17}$ contains up to 21 carbon atoms and is (1) a hydrocarbon radical or (2) an acyl radical of an organic acyclic or monocyclic carboxylic acid containing not more than 1 heteroatom in the ring;

r an q are each independently 0-20 provided that the total of r and q does not exceed 20; and

p is 0 or 1;

$R_3$ is alkyl of 1 to 6 carbon atoms which may be straight chain or branched; or alkenyl of 3 to 6 carbon atoms which may be straight chain or branched as illustrated in Formulae IV and V;

$R_9$ is alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched; $R_4\overset{O}{\overset{\|}{C}}-$ or $R_4OCH_2-$;

and a pharmaceutically acceptable salt or acid addition salt thereof.

As used herein, the terms "each independently" or the equivalents thereof are employed to describe a number of possible position isomers and/or structural variations. For example, as described above, $R_2$ is:

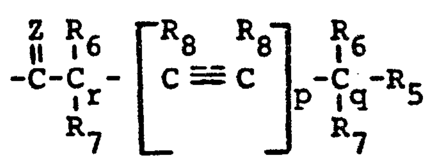

The letters r and q, represent possible alkane chains of from 0-20 carbon atoms, each having the $R_6$ and $R_7$ substituent groups. On each carbon atom of the alkane chain, the $R_6$ and/or $R_7$ substituent may be different. The above description therefore contemplates structures such as the following for the segments $-(CR_6R_7)_r-$ and $-(CR_6R_7)_q-$:

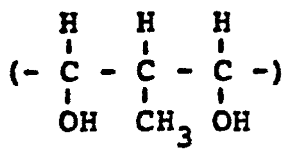 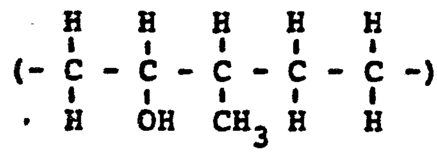

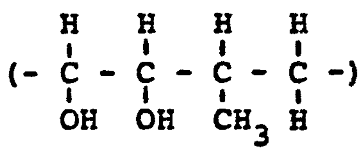 and the like.

The compounds of the present invention may be prepared by several different routes. According to one method a compound of Formula I is reacted with an optionally alkyl substituted alkenyl halide of Formula II wherein X is halogen and each $R_6$ is independently H or alkyl of 1-4 carbon atoms to yield the corresponding 2-hydroxy-4-alkenyloxy-acetophenone of Formula III. The compound of Formula III is then subjected to a Claisen rearrangement to yield a 2,4-dihydroxy-3-alkenyl-acetophenone compound of Formula IV. This rearrangement occurs on heating the compound of Formula III either neat or in a high boiling solvent, such as a halogenated hydrocarbon, e.g., dichlorobenzene, at from about 160°to about 210°C. The double bond in the compound of Formula IV may then be reduced, e.g., by catalytic hydrogenation with a catalyst such as Pd/C, to yield the corresponding saturated compound of Formula V.

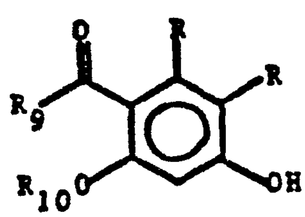 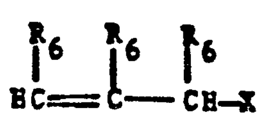 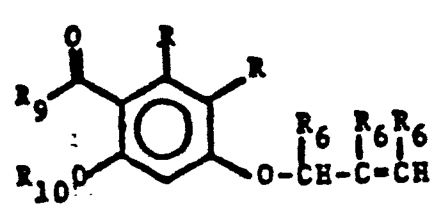

I II III

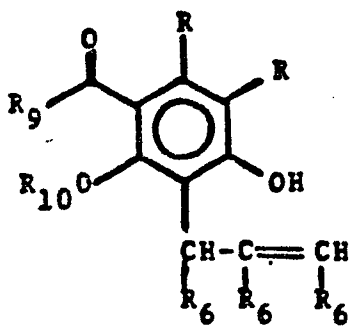

IV

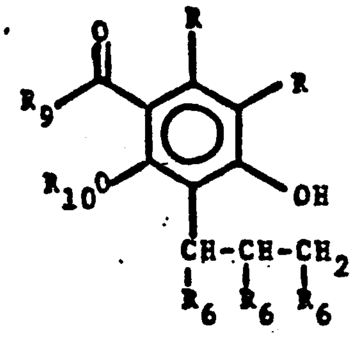

V

The compound of Formula V is then reacted with a dihaloalkane of Formula VIa or a dihaloalkene of Formula VIb wherein X, R and m have the meaning given previously, to yield a 4-(haloalkyloxy)-3-alkyl-2-hydroxyacetophenone compound of Formula VII. The reaction takes place by refluxing a mixture of the compounds of Formulae V and VIa or VIb in an inert solvent such as, for example, methylethylketone (MEK), acetone, tetrahydrofuran (THF), triglyme or dichloromethane in the presence of a base. The reflux temperature is preferably in the range of from about 60 to about 130°C. The base may be an alkali metal carbonate, for example, $Li_2CO_3$, $Na_2CO_3$ or $K_2CO_3$.

Specific examples of dihaloalkane compounds of Formula VIa are 1,3-dibromopropane, 2-methyl-1,3-dibromopropane, 2,2-dimethyl-1,3-dibromopropane, 3-chloro-2-chloromethyl-1-propene, 1,3-dibromobutane, 1,4-dibromobutane, 1,5-dibromopentane, 1,6-dibromohexane, 1,7-dibromoheptane, 1,8-dibromooctane, 1,9-dibromononane, 1,10-dibromodecane, and 1,12-dibromododecane. A specific example of a dihaloalkene compound of Formula VIb is 1,4-dibromo-2-butene.

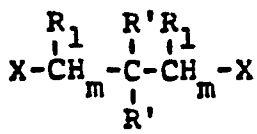

VIa

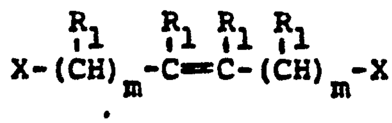

VIb

VII

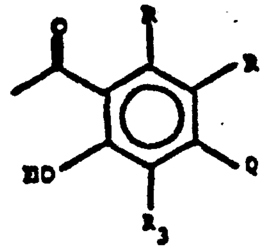

wherein Q is

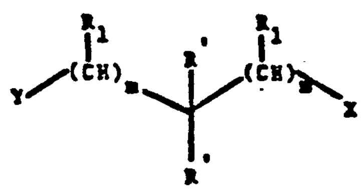

or

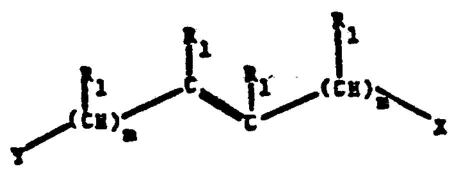

In lieu of the dihaloalkane or dihaloalkene, the compound of Formula IV or V may be reacted with an epihalohydrin, e.g., epichlorohydrin, under the same conditions to yield the 4-(2,3-epoxypropyloxy)-3-alkyl or 3-alkenyl-2-hydroxy-acetophenone compound of Formula VIII.

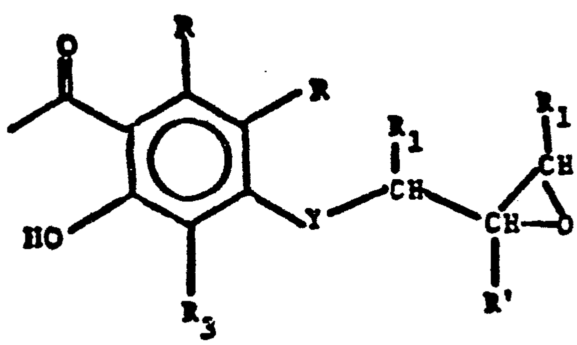

VIII

An alternative procedure is to react a compound of Formula II with a compound of Formula V to yield a 4-alkenyloxy-3-alkyl-2-hydroxy-acetophenone compound of Formula IX which is then epoxidized with an organic peracid such as, for example, m-chloroperbenzoic acid to give the compound of Formula VIII.

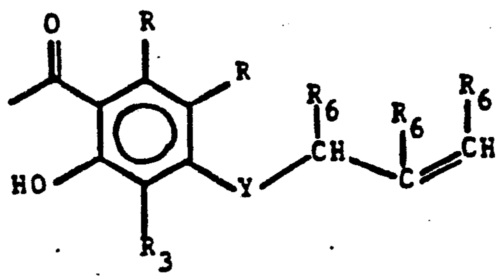

IX

The reaction of a compound of Formula VII with a compound of Formula X, under the same conditions used to react a compound of Formula V with a compound of Formula VIa or VIb, gives compounds of Formulae XI or XII.

The reaction of a compound of Formula VIII with a compound of Formula X under the same conditions used to react a compound of Formula V with a compound of Formula VIa or VIb gives a compound of Formula XI wherein each m is 1.

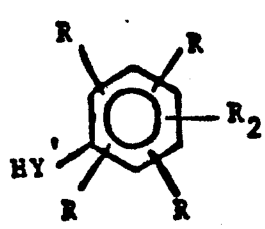

X

When Y' is $-CH_2-$, HY' is methyl. When Y' is $-\overset{O}{\overset{\|}{C}}-$, HY' is a protected aldehyde, such as, for example, a dithioacetal.

When Y' is methylene or carbonyl a stronger base such as, for example, lithium diisopropylamide or butyl lithium is employed in an inert solvent such as tetrahydrofuran with a suitable compound of Formula X.

A compound of Formula X is prepared by subjecting a compound of the Formula XIII:

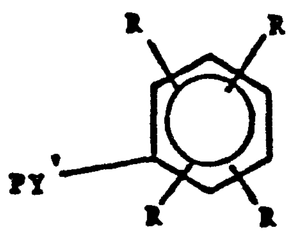

XIII

wherein P is H or a protecting group such as, for example, methyl, benzyl, p-nitrobenzyl or 3-(m-nitrophenyl)-1-phenyl-1-oxo-3-propyl to a Friedel-Crafts reaction with an acyl halide or an acid anhydride. Other compounds of Formulae XI and XII are obtained by reacting a compound of Formula VII with a compound of Formula X under the same conditions used to react a compound of Formula V with a compound of Formula VIa or VIb.

Alternatively, the compounds of Formulae XI and XII may be prepared by reacting a compound of Formula X with a compound of Formula VIa or VIb or an epihalohydrin under the same conditions as described for the preparation of the compound of Formula VII to

give a compound of the Formula XIVa, XIVb or XIVc respectively. The latter are then reacted under the same conditions with a compound of Formula V to give compounds of Formulae XI or XII.

XIVa

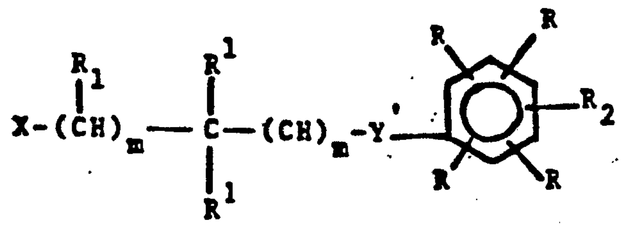

XIVb

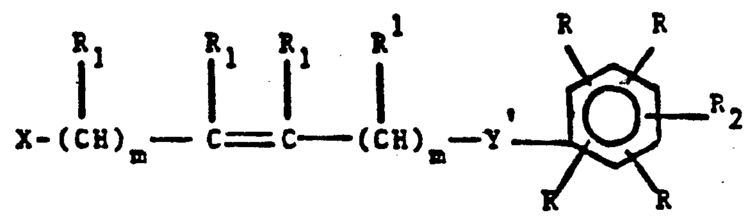

XIVc

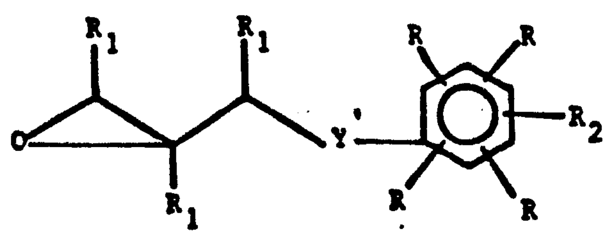

Alternatively, but less preferably, the compounds of Formulae IV or V, X and either VIa, VIb or epihalohydrin may be reacted simultaneously under the conditions described above for reacting a compound of Formula VIa or VIb or epihalohydrin with a compound of Formula X.

Prodrug ester derivatives of the compounds of Formulae XI and XII may be prepared using conventional synthetic techniques available to the skilled artisan. For example, compounds of the Formula XV:

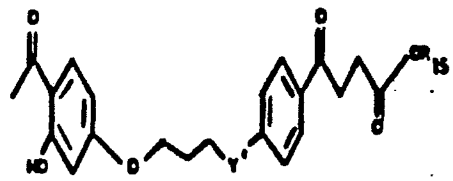

XIV

may be prepared as follows:

Method A:

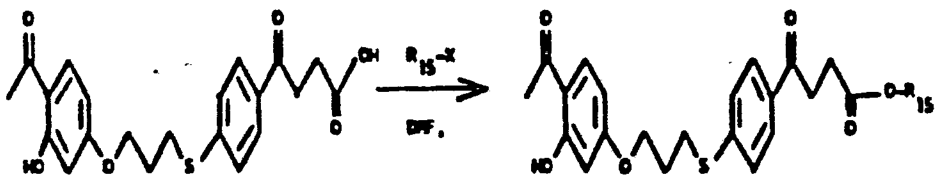


XVI

In Method A, the carboxylic acid of Formula XVI is reacted, in the presence of a base with an alkylhalide compound to provide the prodrug ester.

Method B:

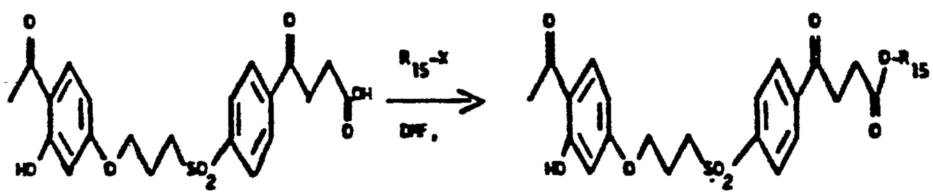


XVII

In Method B, the sulfone carboxylic acid of Formula XVII is similarly reacted with an appropriate alkylhalide, in the presence of base, to provide the corresponding prodrug ester.

Method C:

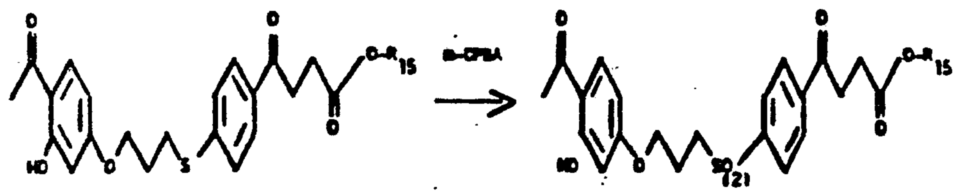


In Method C, the product of the Method A reaction, a produrg ester, may be selectively oxidized to yield the sulfone or the sulfoxide compound.

In structures XI or XII, if Z=H+OH or $R_7$=OH and $R_5$=COOH, a lactone ring may be formed which would act as a prodrug form of the hydroxyacid. For example, a dehydration reaction of a compound having the Formula XVIII:

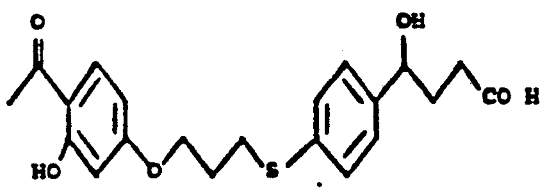

XVIII

such as, by reaction with trifluoroacetic acid, would yield a lactone compound having the Formula XIX:

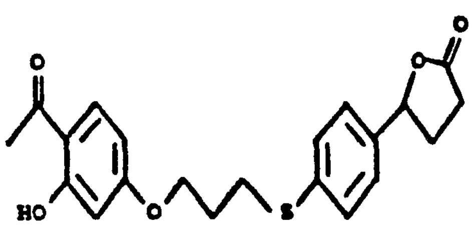

XIX

which when administered orally to a mammal would release the hydroxy acid form.

EXAMPLE 1

4-((3-(4-Acetyl-3-hydroxy-2-propylphenoxy)-2-hydroxypropyl)thio)-gamma-oxobenzenebutanoic acid

A. Preparation of 4-methoxy-gamma-oxobenzenebutanoic Acid

Anisole (70.0 g) and succinic anhydride (65.0 g) were dissolved in 1,2-dichloroethane (1 liter) and the mixture was cooled to 0°C. To the resulting suspension there was added, in portions, $AlCl_3$ (172 g) and the resulting mixture was stirred with a mechanical stirrer for 1 hour. The mixture was then poured into a mixture of ice and water (about 1 liter) containing 50 ml of concentrated HCl. The resulting white solid was collected by filtration, washed with water and air-dried to yield 4-methoxy-gamma-oxobenzenebutanoic acid, mp 145-147°C.

B. Preparation of 4-hydroxy)-gamma-oxobenzenebutanoic acid, methyl ester

A mixture of the compound from Step A (77.3 g), 48% HBr (310 ml), and acetic acid (620 ml) was heated under reflux for 18 hours. The resulting mixture was cooled to room temperature and poured into 3 liters of water. The resulting solution was extracted with ethyl acetate (3 x 500 ml). The combined organic layers were washed with water (4 x 200 ml), dried over $Na_2SO_4$, the solvents were removed by evaporation and the residue was dissolved in 10% HCl/methanol (500 ml). After 1 hour at room temperature the volatile components were removed by evaporation in vacuo. The resulting residue was triturated with hexane to yield the title compound, mp 115-116°.

C. Preparation of 4-dimethylthiocarbamoyloxy-gamma-oxobenzenebutanoic acid, methyl ester

A solution of the product from Step B, 25 g, in anhydrous dimethylformamide (DMF) (300 ml) was cooled to 0° and 99% NaH, 3.46 g, was added in two portions. The mixture was stirred for 1 hour at 0° then dimethylthiocarbamoyl chloride, 19.3 g, was added and the mixture heated at 90° under a $N_2$ atmosphere for 1.5 hours. The mixture was cooled to room temperature and diluted with water to 1,200 mL. The resulting solution was then extracted with ethyl acetate (3 x 500 ml). The combined organic layers

were washed with brine and then dried over $Na_2SO_4$ and evaporated to dryness *in vacuo* to yield a residue which was purified by chromatography on silica gel to yield the title compound, mp 62-64°.

D. Preparation of 4-dimethylcarbamoylthio-gamma-oxobenzenebutanoic acid, methyl ester

The compound from Step C, 29.6 g, was heated at 200° for 10 hours under an $N_2$ atmosphere. The mixture was cooled to room temperature, dissolved in methylene chloride and purified by chromatography on silica gel to provide the title compound, mp 98-100°.

E. Preparation of 4-mercapto-gamma-oxobenzenebutanoic acid, methyl ester

Sodium 280 mg, was dissolved in anhydrous methanol, 50 ml, under an $N_2$ atmosphere. To the resulting solution there was added 5.0 g of the compound from Step D. The mixture was stirred at room temperature overnight, then poured into a mixture containing 30 ml of water and 7 ml of concentrated HCl. The resulting yellow solid was collected by filtration, washed with water and dried in air to give the title compound, mp 83-84°.

F. Preparation of 4-((3-(4-acetyl-3-hydroxy-2-propylphenoxy)-2-hydroxypropyl)thio)-gamma-oxobenzenebutanoic acid

2-Hydroxy-3-propyl-4-(2,3-epoxypropoxy)-acetophenone (0.6 g, 2.4 mmole) was refluxed in 15 ml of methylethyl ketone with the product from step E (0.6 g, 2.4 mmole) for two days in the presence of 1.0 equivalent of $K_2CO_3$. The product was purified by chromatography on silica gel. The product, 0.9 g was saponified with KOH (1.5 equivalents) in a 25 ml mixture of methanol and water

(10:1). The volatile components were removed under vacuum. The residue was taken up in water and acidified with citric acid. The aqueous phase was extracted twice with ethyl acetate, the organic layer was washed with brine, dried ($Na_2SO_4$) and evaporated to dryness to leave the acid residue which was triturated in hexane and filtered.

Analysis, Calculated: C, 62.18; H, 6.74.
Observed: C, 62.14, H, 6.32.

EXAMPLE 2

4-((3-(4-Acetyl-3-hydroxy-2-propylphenoxy)-2-hydroxypropyl)thio)-2-fluoro-gamma-oxobenzenebutanoic acid

Following the procedure of Example 1 but substituting an equivalent amount of 3-fluoroanisole for anisole in Step A, the title compound was obtained, mp 136-137°.

EXAMPLE 3

4-((3-(4-Acetyl-3-hydroxy-2-propylphenoxy)-2-hydroxypropyl)thio)-3-fluoro-gamma-oxobenzenebutanoic acid

Following the procedure of Example 1 but substituting an equivalent amount of 2-fluoroanisole for anisole in Step A, the title compound was obtained, m.p. 106-109°C.

EXAMPLE 4

Methyl 4-((3-(4-acetyl-3-hydroxy-2-propylphenoxy)--2-hydroxypropyl)thio)-2-hydroxy-gamma-oxobenzene butanoate

A. Preparation of 3-(3-nitrophenyl)-3-(3-methoxy-phenylthio)-1-phenyl-1-propanone

Piperidine, 1.2 ml, was added to a boiling solution of 3-nitrobenzalacetophenone (10 g, 0.04 mole) and 3-methoxybenzenethiol (6.64 g, 0.048 mole) in 100 ml benzene . After standing for 30 minutes without further heating, acetic acid, 10 ml, was added. The solution then was poured into water (about 100 ml), extracted with methylene chloride (5 x 100 ml), washed with water, dried ($Na_2SO_4$) and evaporated to dryness to yield the title compound as a solid, mp 105°.

B. Preparation of 4-(3-phenyl-3-oxo-1-(3-nitrophenyl)-1-propylthio)-2-hydroxy-gamma-oxobenzenebutanoic acid, methyl ester

Succinic anhydride (11.45 g, 0.11 mole) was dissolved in 1,2-dichloroethane (300 ml) and $AlCl_3$ (50.76 g, 0.38 mole) was added under $N_2$. The resulting mixture was stirred for 3 hours at room temperature, cooled to 0°C and the compound from Step A (13 g, 0.03 mole), dissolved in 1,2-dichloroethane, was added slowly. The reaction mixture was kept at 5°C for 3 days, then poured on ice, stirred for one hour and the two phases separated by decanting. The aqueous phase was extracted several times with $CH_2Cl_2$ (600 ml total). The organic phases were combined, washed with water, dried ($MgSO_4$) and evaporated to dryness to yield the title compound as the free acid which then was converted to the methyl ester in conventional manner by treating the acid with methanol and anhydrous HCl. The ester had the following analysis: calculated: C, 63.27; H, 4.70; S, 6.50; observed: C, 63.19; H, 4.81; S, 6.63.

C. Preparation of 4-mercapto-2-hydroxy-gamma-oxobenzenebutanoic acid, methyl ester

To a well stirred solution of the ester from Step B (1.0 g, 2.02 mmoles) in a mixture of $CHCl_3$ (20 ml) and $C_2H_5OH$ (20 ml) was added lead acetate trihydrate (6 equivalents). After stirring the solution for 15 minutes, 10% NaOH was added to maintain the pH at 9-10. A yellow precipitate formed. After stirring for three hours, the suspension was filtered and washed with $CHCl_3$. The solid was taken up in methanolic HCl (10 ml), stirred for one hour and evaporated to dryness. The residue was taken up in ethyl acetate, the white solid was filtered off and the filtrate evaporated to dryness to yield the title compound calculated: C, 54.98, H, 5.04, S, 13.35; observed: C, 55.10, H, 4.94, S, 13.21.

D. Preparation of methyl 4-((3-(4-acetyl-3-hydroxy-2-propylphenoxy)-2-hydroxypropyl) thio)-2-hydroxy-gamma-oxobenzenebutanoate

Following the procedure of Step F of Example 1 but substituting an equivalent amount of the ester from Step C above for 4-mercapto-gamma-oxobenzenebutanoic acid, methyl ester, the title compound was obtained.

Analysis, calculated: C, 61.21; H, 6.16.
Observed: C, 61.13; H, 6.30.

EXAMPLE 5

4-((3-(4-Acetyl-3-hydroxy-2-propylphenoxy)-2-hydroxypropyl)thio)-2-hydroxy-gamma-oxobenzenebutanoic acid

The compound prepared according to Example 4 (544 mg, 1.15 mmol) was refluxed with $NaHCO_3$ (482 mg, 5.75 mmole) in a 20 ml mixture of methanol and water (3:1) for 4 hours. Then in hydrochloric acid

was added and the mixture was extracted with $CH_2Cl_2$, washed with water, dried ($Na_2SO_4$), and evaporated to dryness to yield a residue which was recrystallized from methanol/hexane, mp 149.5°. Analysis, calculated: C, 62.45; H, 6.33; S, 6.95. Observed: C, 62.46, H, 6.37; S, 7.24.

EXAMPLE 6

4-((3-(4-Acetyl-3-hydroxy-2-propylphenoxy)propyl)-thio)-gamma-oxobenzenebutanoic acid

Following the procedure of Step F of Example 1 but substituting an equivalent amount of 2-hydroxy-3-propyl-4-(3-bromopropyloxy)acetophenone for 2-hydroxy-3-propyl-4-(2,3-epoxypropoxy)-acetophenone, the title compound was obtained. Analysis, calculated: C, 64.98; H, 6.14; S, 7.23. Observed: C, 65.09; H, 6.09; S, 7.28.

EXAMPLE 7

4-((3-(4-Acetyl-3-hydroxyphenoxy)propylthio)-gamma-oxobenzenebutanoic acid

Following the procedure of Step F of Example 1, but substituting 2-hydroxy-4-(3-bromopropyloxy) acetophenone for 2-hydroxy-3-propyl-4-(2,3-epoxy-propoxy)acetophenone, the title compound was obtained. Analysis, calculated: C, 62.66; H, 5.51; S, 7.96. Observed: C, 62.72; H, 5.64; S, 7.90.

EXAMPLE 8

4 ((3-(4-Acetyl-3-hydroxy-2-propylphenoxy)propyl)-thio)-2-hydroxy-gamma-oxobenzenebutanoic acid

A. Preparation of methyl ester of 4-((3-(4-acetyl-3-hydroxy-2-propylphenoxy)propyl)thio)-2-hydroxy-gamma-oxobenzenebutanoic acid

Following the procedure of Step F of Example 1 but substituting an equivalent amount of 2-hydroxy-3-propyl-4-(3-bromopropyloxy)acetophenone for 2-hydroxy-3-propyl-4-(2,3-epoxypropoxy)acetophenone and substituting an equivalent amount of the product from Step C of Example 4 for 4-mercapto-gamma-oxobenzenebutanoic acid, methyl ester, the title compound was obtained.

Analysis, calculated: C, 63.27; H, 6.37; S, 6.76.
Observed: C, 63.30; H, 6.54; S, 6.70.

B. Preparation of 4-((3-(4-acetyl-3-hydroxy-2-propylphenoxy)propyl)thio)-2-hydroxy-gamma-oxobenzenebutanoic acid

By treating the product from Step A of this example according to the procedure of Example 5, the title compound was obtained, mp 140°C.

Analysis, calculated: C, 63.27; H, 6.37; S, 6.76.
Observed: C, 63.30; H, 6.54; S, 6.70.

## EXAMPLE 9

4-((3-(4-Acetyl-3-hydroxy-2-propylphenoxy)-2-hydroxypropyl)thio)-2-methoxy-gamma-oxobenzenebutanoic acid

A. Preparation of 4-mercapto-2-methoxy-gamma-oxobenzenebutanoic acid, methyl ester

The free acid from Step B of Example 4 was dissolved in $CH_3OH$ and treated with excess diazomethane at room temperature. Volatiles were removed under vacuum. After purification on silica gel, there was obtained the methyl ester of 4-(3-phenyl-3-oxo-1-(m-nitrophenyl)-1-propylthio)-2-methoxy-gamma-oxobenzenebutanoic acid. This ester was reacted with lead acetate trihydrate to yield

4-mercapto-2-methoxy-gamma-oxobenzenebutanoic acid. The methyl ester of the acid was obtained in conventional manner by treatment with methanolic HCl.

B. Preparation of 4-((3-(4-acetyl-3-hydroxy-2-propylphenoxy)-2-hydroxypropyl)thio)-2-methoxy-gamma-oxobenzenebutanoic acid

Following the procedure of Step F of Example 1 but substituting an equivalent amount of the ester from Step A of this example for 4-mercapto-gamma-oxobenzenebutanoic acid, methyl ester, the title compound was obtained.

Analysis, calculated: C, 61.20; H, 6.16; S, 6.53.
Observed: C, 61.14; H, 6.26; S, 6.51.

EXAMPLE 10

4-((3-(4-Acetyl-3-hydroxy-2-propylphenoxy)propyl)-thio)-3-fluoro-gamma-oxobenzenebutanoic acid

Following the procedure of Example 1 but substituting an equivalent amount of 2-fluoroanisole for anisole in Step A and substituting an equivalent amount of 2-hydroxy-3-propyl-4-(3-bromopropoxy)-acetophenone for 2-hydroxy-3-propyl-4-(2,3-epoxypropoxy)acetophenone in Step F, the title compound was obtained, m.p. 115-116°C.

EXAMPLE 11

4-((3-(4-Acetyl-3-hydroxy-2-propylphenoxy)propyl)-thio)-2-fluoro-gamma-oxobenzenebutanoic acid

Following the procedure of Example 10 but substi·uting 3-fluoroanisole for 2-fluoroanisole, the title compound was obtained, mp 149-150°C.

## EXAMPLE 12

**4-(3-(4-Acetyl-3-hydroxy-2-propylphenoxy)propoxy)-3-fluoro-gamma-oxobenzenebutanoic acid**

A. Preparation of 4-Hydroxy-3-fluoro-gamma-oxobenzenebutanoic acid, methyl ester

To a solution of 2-fluorophenol (22.4 g, 200 mmole) in 1,2-dichloroethane (250 ml) at 0° there was added $AlCl_3$ (54 g, 400 mmole) and then succinic anhydride (20 g, 200 mmole). The reaction mixture was heated at 85° C for 18 hours, then cooled, poured on ice and stirred for several hours at room temperature. The layers were separated and the aqueous layers extracted with $CH_2Cl_2$. The combined extracts were washed with water, dried ($MgSO_4$) and evaporated to a semi-solid which was taken up in a solution of methanolic HCl (500 ml, approximately 3N). After standing for 24 hours the solution was poured into water, extracted with $CH_2Cl_2$, washed with water, dried and evaporated to a small volume. The crude product was purified by chromatography on silica gel to yield the title compound.

Analysis, calculated: C, 58.41; H, 4.91; F, 8.40. Observed: C, 58.63;, H, 4.96; F, 8.14. Its isomer, 2-hydroxy-3-fluoro-gamma-oxobenzenebutanoic acid, methyl ester, was also isolated in the chromatographic step.

Analysis, calculated: C, 58.41; H, 4.91; F, 8.40. Observed: C, 58.53; H, 4.82; F, 8.10.

B. Preparation of 4-(3-(4-acetyl-3-hydroxy-2-propylphenoxy)propoxy)-3-fluoro-gamma-oxobenzenebutanoic acid

Following the procedure of Example 6 but substituting the title ester from Step A of this example for 4-mercapto-gamma-oxobenzenebutanoic acid, methyl ester, the title compound was obtained, m.p. 142-144°C.

Analysis, calculated: C, 64.56; H, 6.10; F, 4.26.
Observed: C, 64.54, H, 6.32; F, 4.05.

C. Preparation of 2-(3-(4-acetyl-3-hydroxy-2-propylphenoxy)propoxy)-3-fluoro-gamma-oxobenzenebutanoic acid

Following the procedure of Step B of this example but substituting 2-hydroxy-3-fluoro-gamma-oxobenzenebutanoic acid, methyl ester for 4-hydroxy-3-fluoro-gamma-oxobenzenebutanoic acid, methyl ester, the title compound was obtained.

Analysis, calculated: C, 64.56; H, 6.10; F, 4.26.
Observed: C, 64.54, H, 6.32; F, 4.05.

## EXAMPLE 13

4-(3-(4-Acetyl-3-hydroxy-2-propylphenoxy)propoxy) -3-fluoro-epsilon-oxobenzenehexanoic acid

A. Preparation of 4-hydroxy-3-fluoro-epsilon-oxobenzenehexanoic acid, methyl ester

Following the procedure of Example 12 but adding $TiCl_4$ (2.5 ml, 21.8 mmole) at -15°C in lieu of $AlCl_3$ in Step A and substituting methyl 5-chloroformylpentanoate (3.57 g, 20 mmole) for succinic anhydride, and then heating the reaction mixture to 90°C for 3 hours, the title compound was obtained.

Analysis, calculated: C, 61.51; H,, [illegible].45; F, 7.47.
Observed: C, 61.51; H, 6.14; F, 7.08. Its isomer, 2-hydroxy-3-fluoro- -epsilon-oxobenzenehexanoic acid,

methyl ester, was also obtained, m.p. 59-60°C.
Analysis, calculated: C, 61.41; H, 5.95; F, 7.47.
Observed: C, 61.58; H, 6.05; F, 7.17.

B. Preparation of 4-(3-(4-acetyl-3-hydroxy-2-propylphenoxy)propoxy)-3-fluoro-epsilon-oxobenzenehexanoic acid

Following the procedure of Step B of Example 12, but substituting the title ester from Step A of this example for 4-hydroxy-3-fluoro-gamma-oxobenzenebutanoic acid, methyl ester, the title compound was obtained, m.p. 106-107°C.
Analysis, calculated: C, 65.81; H, 6.59; F, 4.00.
Observed: C, 65.90; H, 6.18; F, 3.71.

C. Preparation of 2-(3-(4-acetyl-3-hydroxy-2-propylphenoxy)propoxy)-3-fluoro-epsilon-oxobenzenehexanoic acid

Following the procedure of step B of this example but substituting 2-hydroxy-3-fluoro-epsilon-oxobenzenehexanoic acid, methyl ester for the title compound of Step A of this example, the title compound was obtained.
Analysis, calculated: C, 65.81; H, 6.53; F, 4.00.
Observed: C, 65.90; H, 6.13; F, 3.71.

EXAMPLE 14

4-(3-(4-Acetyl-3-hydroxy-2-propylphenoxy)-propoxy)-2-fluoro-gamma-oxobenzenebutanoic acid

Following the procedure of Steps A and B Example 12 but substituting an equivalent amount of 3-fluorophenol for 2-fluorophenol in Step A, the title compound was obtained, m.p. 113-118°C.

## EXAMPLE 15

4-(3-(4-Acetyl-3-hydroxy-2-propylphenoxy)-propoxy)-gamma-oxobenzenebutanoic acid

By reacting the compound of Step B of Example 1 with 2-hydroxy-3-propyl-4-(3-bromo-propyloxy)acetophenone according to the procedure of Step F of Example 1, the title compound was obtained.

Analysis, calculated: C, 67.27; H, 6.58.
Observed: C, 67.30; H, 6.56.

## EXAMPLE 16

4-(3-(4-Acetyl-3-hydroxy-2-propylphenoxy)propoxy)-2,3-dichloro-gamma-oxobenzenebutanoic acid

Following the procedure of Steps A and B of Example 1, but substituting 2,3-dichloroanisole for anisole in Step A, there was obtained 2,3-dichloro-4-hydroxy-gamma-oxobenzenebutanoic acid, methyl ester. The ester was reacted with 2-hydroxy-3-propyl-4-(3-bromo-propyloxy)acetophenone according to the procedure of Step F of Example 1 to give the title compound, mp 119-123°C.

## EXAMPLE 17

4-(3-(4-Acetyl-3-hydroxy-2-propylphenoxy)propoxy)-3-methyl-gamma-oxobenzenebutanoic acid

Following the procedure of Example 16, but substituting 2-methyl anisole for 2,3-dichloro anisole there was obtained 3-methyl-4-hydroxy--gamma-oxobenzenebutanoic acid, methyl ester. The ester then was reacted with 2-hydroxy-3-propyl-4-(3-bromopropyloxy)acetophenone according to the procedure of Step F of Example 1 to give the title compound, mp 179-180°C.

Analysis, calculated: C, 67.86; H, 6.83.
Observed: C, 6.83; H, 6.80.

EXAMPLE 18

4-(3-(4-Acetyl-3-hydroxy-2-propylphenoxy)propoxy)-2-chloro-gamma-oxobenzenebutanoic acid

Following the procedure of Example 16 but substituting 3-chloroanisole for 2,3-dichloroanisole, there was obtained 2-chloro-4-hydroxy-gamma-oxobenzenebutanoic acid, methyl ester. The ester then was reacted with 2-hydroxy-3-propyl-4-(3-bromopropyloxy)acetophenone according to the procedure of Step F of Example 1 to give the title compound, mp 143-144.5°C.

Analysis, calculated: C, 62.27; H, 5.88; Cl, 7.66.
Observed: C, 62.14; H, 5.92; Cl, 7.89.

EXAMPLE 19

4-(4-(4-Acetyl-3-hydroxy-2-propylphenoxy)-2-butenoxy)-3-fluoro-gamma-oxobenzenebutanoic acid

Following the procedure of Step F of Example 1 but substituting 2-hydroxy-3-propyl-4-(4-chloro-2-butenoxy)acetophenone for 2-hydroxy-3-propyl-4-(2,3-epoxypropoxy)acetophenone, and substituting 4-hydroxy-3-fluoro-gamma-oxobenzenebutanoic acid, methyl ester for the methyl ester product of Step E of Example 1, the title compound was obtained, mp 170-171.5°C.

Analysis, calculated: C, 65.49; H, 5.94; F, 4.34.
Observed: C, 65.58; H, 6.02; F, 4.22.

EXAMPLE 20

4-(3-(4-acetyl-3-hydroxy-2-propylphenoxy)propoxy-3-propyl-gamma-oxobenzenebutanoic acid

Following the procedure of Example 17 but substituting 2-n-propyl anisole for 2-methylanisole, the title compound was obtained.

Analysis, calculated: C, 67.27; H, 6.58.

Observed: C, 67.30; H, 6.54.

EXAMPLE 21

4-(3-(4-Acetyl-3-hydroxy-2-propylphenoxy)-2-hydroxypropylthio)-3-fluoro-gamma-oxobenzenebutanoic acid-S-oxide

The ester prepared according to the procedure of Example 3 was dissolved in 50 mL $CH_2Cl_2$, the solution was cooled to 0° and 1 equivalent of m-chloroperbenzoic acid was added. The reaction mixture was stirred at 0°C for 5 minutes and was purified by chromatography on silica gel to yield the methyl ester of the title compound, that was saponified by stirring with sodium hydroxide in methanol to yield the title compound, m.p. 179-181°C.

EXAMPLE 22

4-(3-(4-Acetyl-3-hydroxy-2-propylphenoxy)-propylthio)-3-fluoro-gamma-oxobenzenebutanoic acid-S-oxide

The ester prepared according to the procedure of Example 10 was treated as in Example 21 to give, after saponification, the title compound, m.p. 135-135°C.

EXAMPLE 23

4-(3-(4-Acetyl-3-hydroxy-2-propylphenoxy)-propylthio)-2-fluoro-gamma-oxobenzenebutanoic acid-S-oxide

The ester prepared according to the procedure of Example 11 was treated as in Example 21 to give, after saponification, the title compound, m.p. 168-169°C.

EXAMPLE 24

4-(3-(4-Acetyl-3-hydroxy-2-propylphenoxy)-2-hydroxypropylthio)-2-fluoro-gamma-oxobenzenebutanoic acid-S-oxide

The ester prepared according to the procedure of Example 2 was treated as in Example 21 to give, after saponification, the title compound, m.p. 69-72°C.

EXAMPLE 25

4-(3-(4-Acetyl-3-hydroxy-2-propylphenoxy)propylsulfonyl)-2-fluoro-gamma-oxobenzenebutanoic acid

The ester prepared according to the procedure of Example 11 dissolved in 50 mL $CH_2Cl_2$ was treated at room temperature with 2.5 equivalents of m-chloroperbenzoic acid for 2 hours. The reaction mixture was purified by chromatography on silica gel to yield the methyl ester of the title compound, that then was saponified by stirring with NaOH in methanol to yield the title compound, m.p. 116-118°.

EXAMPLE 26

4-(3-(4-Acetyl-3-hydroxy-2-propylphenoxy)-2-hydroxypropylsulfonyl)-2-fluoro-gamma-oxobenzenebutanoic acid

The ester prepared according to the procedure of Example 2 was treated as in Example 25 to give, after saponification, the title compound, m.p. 80-85° (decomp).

EXAMPLE 27

4-(3-(4-Acetyl-3-hydroxy-2-propylphenoxy)-propylsulfonyl)-3-fluoro-gamma-oxobenzenebutanoic acid

The ester prepared according to the procedure of Example 10 was treated as in Example 25 to give, after saponification, the title compound, m.p. 154-156°C.

EXAMPLE 28

4-(3-(4-Acetyl-3-hydroxy-2-propylphenoxy)-2-hydroxypropylsulfonyl)-3-fluoro-gamma-oxobenzenebutanoic acid

The ester prepared according to the procedure of Example 3 was treated as in Example 25 to give, after saponification, the title compound, m.p. 96-99° resolidifies, 105-108°.

EXAMPLE 29

4-(3-(4-Acetyl-3-hydroxy-2-propylphenoxy)-propylsulfonyl)-gamma-oxobenzenebutanoic acid

The ester prepared according to the procedure of Example 6 was treated as in Example 25 to give, after saponification, the title compound, Analysis, calculated: C, 60.48; H, 5.92; S, 6.72. Observed: C, 60.51; H, 5.90; S, 6.48.

EXAMPLE 30

4-(3-(4-Acetyl-3-hydroxy-2-propylphenoxy)-2-hydroxypropylsulfonyl)-gamma-oxobenzenebutanoic acid

The ester prepared according to the procedure of Example 1 was treated as in Example 25 to give, after saponification, the title compound.

Analysis, calculated: C, 59.27; H, 5.97; S, 6.33.
Observed: C, 59.22; H, 6.13; S, 6.02.

EXAMPLE 31

4-(3-(4-Acetyl-3-hydroxy-2-propylphenoxy)-propoxy)-3-chloro-gamma-oxobenzenebutanoic acid

By following the procedure of Example 12 but substituting in Step A, 2-chlorophenol for 2-fluorophenol, the title compound is obtained, m.p. 167.5-169.5°

EXAMPLE 32

4-(3-(4-Acetyl-3-hydroxy-2-propylphenoxy)-2-hydroxypropoxy)-3-fluoro-gamma-oxobenzenebutanoic acid, sodium salt, monohydrate

Following the procedure of Step F of Example 1 but substituting an equivalent amount of 2-hydroxy-3-propyl-4-(3-bromopropyloxy)acetophenone for 2-hydroxy-3-propyl-4-(2,3-epoxypropoxy)-acetophenone and substituting the product from Step A of Example 12 for 4-mercapto-gamma-oxobenzenebutanoic acid, methyl ester, the corresponding free acid of the title compound was isolated. It was converted to the sodium salt by treating with 1 equivalent of NaOH.

Analysis, calculated: C, 57.42; H, 5.81; F, 3.78.
Observed: C, 57.49; H, 5.78; F, 4.19.

EXAMPLE 33

4-(3-(4-Acetyl-3-hydroxy-2-propylphenoxy)-propoxy)-alpha,alpha-dimethyl-gamma-oxobenzenebutanoic acid

A: Preparation of 4-3,4-dehydro-2,2-dimethyl-4-(4-methoxyphenyl)-gamma-butyrolactone

To a stirring suspension of KH (16 mmole) in THF (50 ml) was added a solution of 4-methoxy-gamma-oxobenzenebutanoic acid (8 mmole) prepared as in Step A of Example 1 in THF (10 ml) at room temperature. The mixture was stirred at room temperature for 2 hours. Methyl iodide (32 mmole) was added and the resulting reaction mixture stirred at room temperature for 16 hours. Cold, dilute HCl solution was added and the resulting mixture extracted with ethyl acetate. The organic layer was washed successively with $NaHCO_3$ solution, brine, dried and evaporated.

The resulting oil was chromatographed on silica gel to give 3,4-dehydro-2,2-dimethyl-4-(4-methoxyphenyl)-gamma-butyrolactone.

B: Preparation of methyl 4-methoxy-alpha,alpha-dimethyl-gamma-oxobenzenebutanoate

To 3,4-dehydro-2,2-dimethyl-4(4-methoxyphenyl)-gamma-butyrolactone (1.35 mmole), prepared in Step A of this Example, in methanol (8 ml) was added 2.7 mmole of sodium methoxide. The reaction was stirred at room temperature for 1.5 hour. Cold dilute hydrochloric acid was then added, the methanol evaporated to dryness and the resulting aqueous layer extracted with ethyl acetate. The organic layer was then washed with brine and dried over $MgSO_4$. Evaporation of the volatiles gave a quantitive yield of the title compound.

C: Preparation of 4-(3-(4-Acetyl-3-hydroxy-2-propylphenoxy)propoxy)-alpha,alpha-dimethyl-oxobenzenebutanoic acid

Following the procedure of Step B of Example 1 but substituting the product from Step B of this example for 4-methoxy-gamma-oxobenzenebutanoic acid there was obtained 4-hydroxy-alpha,alpha-dimethyl-gamma-oxobenzenebutanoic acid, methyl ester.

This ester was reacted according to the procedure of Example 6 to yield the title compound. Analysis, calculated: C, 68.40; H, 7.07. Observed: C, 68.54; H, 7.06.

EXAMPLE 34

4-(3-(4-Acetyl-3-hydroxy-2-propylphenoxy)propoxy)-beta-methyl-gamma-oxobenzenebutanoic acid

The title compound was prepared by following the procedure of Step C of Example 33 but substituting the methyl 4-methoxy-beta-methyl-gamma-oxobenzene-butanoate (prepared as described in Step A of Example 33) for the product of Step B of Example 33. Analysis, calculated: C, 67.86; H, 6.83. Observed: C, 67.94, H, 6.87.

EXAMPLE 35

4-(3-(4-Acetyl-3-hydroxy-2-propylphenoxy)-2-methyl-idenylpropoxy-gamma-oxobenzenebutanoic acid

A: Preparation of 2-Hydroxy-3-propyl-4-(3-chloro-2-methylidenylpropoxy)acetophenone

To a olution of 2,4-dihydroxy-3-propyl-acetophenone, .0 g, in acetone, 600 ml, were added $K_2CO_3$, 64 g, and 3-chloro-2-chloromethyl-1-propene, 54 ml. The heterogeneous purple mixture was

stirred at reflux overnight. The reaction mixture was filtered, the solids were washed with acetone and the filtrate was evaporated to a red oil that was chromatographed on silica gel to yield the title compound, mp 50-52°C as a white solid.

B: Preparation of 4-(3-(4-acetyl-3-hydroxy-2-propylphenoxy)-2-methylidenylpropoxy)-gamma-oxo-benzenebutanoic acid

Following the procedure of Step F of Example 1, but substituting the title compound of Step A of this example for 2-hydroxy-3-propyl-4-(2,3-epoxy-propoxy)acetophenone and substituting 4-hydroxy-gamma-oxobenzenebutanoic acid, methyl ester for 4-mercapto-gamma-oxobenzenebutanoic acid, methyl ester, the title compound was obtained, mp 136-137°C. Analysis, calculated: C, 68.17; H, 67.41. Observed: C, 68.10; H, 6.40.

EXAMPLE 36

4-(3-(4-acetyl-3-hydroxy-2-propylphenoxy)-2-methyl-propoxy)-gamma-oxobenzenebutanoic acid

A: Preparation of 2-Hydroxy-3-propyl-4-(2-methyl-3-chloro-propoxy)acetophenone

Following the procedure of Step A of Example 35 but substituting 2-methyl-1,3-dichloropropane for 3-chloro-2-chloromethyl-1-propene, the title compound was obtained.

B: Preparation of 4-(3-(4-Acetyl-3-hydroxy-2-propylphenoxy)-2-methylpropoxy)-gamma-oxobenzenebutanoic acid

Following the procedure of Step B of Example 35, but substituting 2-hydroxy-3-propyl-4-(2-methyl-3-chloro-propoxy)acetophenone for 2-hydroxy-3-propyl-4-(-3-chloro-2-methylidenylpropoxy)acetophenone, the title compound was obtained.

Analysis, calculated: C, 67.86; H, 6.83.
Observed: C, 67.62; H, 6.85.

EXAMPLE 37

4-(3-(4-acetyl-3-hydroxy-2-propylphenoxy)-2-methylidenepropoxy)-gamma-oxo-benzenebutanoic acid

A: Preparation of 2-hydroxy-3-propyl-4(3-chloro-2-methylidenepropoxy)acetophenone

2,4-Dihydroxy-3-propyl-acetophenone (30 g, 15.5 mmole) was refluxed with 3-chloro-2-chloromethyl-1-propene (54 ml, 464 mmole) in 600 ml acetone for 14 hours in the presence of 3.0 equivalents of $K_2CO_3$. The product was purified by chromatography on silica gel using hexane/EtOAc (10:1 - 10:5) as eluent. The title compound was obtained as a white solid, m.p. 50-52°.

B: Preparation of methyl 4-(3-(4-acetyl-3-hydroxy-2-propylphenoxy)-2-methylidenepropoxy)-gamma-oxo-benzenebutanoate

To a stirred mixture of the compound from Step A (6.22 g, 22.0 mmole) in methylethyl ketone (110 ml) was added the compound of Example 1, Step B (5.54 g, 26.6 mmole) and 3.0 equivalents of $K_2CO_3$. The mixture was heated at reflux for 30 hours. The reaction mixture was cooled to room temperature, filtered, washed with acetone and evaporated to dryness. The residue was dissolved in

methylene chloride, washed with 0.1N NaOH, and evaporated to an oil. The oil was purified by HPLC using hexane: EtOAc (10:4) to yield the title compound as a white solid, m.p. 89-90°.
Analysis, calculated: C, 68.70; H, 6.65.
Observed: C, 68.68; H, 6.70.

C: 4-(3-(4-acetyl-3-hydroxy-2-propylphenoxy)-2-methylidenepropoxy)-gamma-oxo-benzenebutanoic acid

To a stirred solution of the compound from Step B (550 mg, 1.21 mmole) in THF (15 ml) was added 3 ml of 1N NaOH. The mixture was stirred at room temperature for 1.5 hours. The reaction mixture was diluted with $H_2O$ (50 ml) and acidified with concentrated HCl to pH=5. A white solid formed which was extracted with methylene chloride (2X). The combined organic extracts were washed with brine, dried ($MgSO_4$) and concentrated *in vacuo* to yield the title compound as a white solid, m.p. 136-137°.
Analysis, calculated: C, 68.17; H, 6.41.
Observed: C, 68.10; H, 6.40.

## EXAMPLE 38

4-(3-(4-acetyl-3-hydroxy-2-propylphenoxy)-2-methylidenepropylthio)-gamma-oxo-benzenebutanoic acid

Following the procedure of Example 37, Steps B and C but substituting an equivalent amount of the compound of Example 1, Step E (for the compound of Example 1, Step B), in Step B of Example 37, the title compound m.p. 123-125° was obtained following hydrolysis of its corresponding methyl ester.
Analysis, calculated: C, 65.77; H, 6.18; S, 7.02.
Observed: C, 65.74; H, 6.23; S, 7.23.

## EXAMPLE 39

4-(3-(4-acetyl-3-hydroxy-2-propylphenoxy)-2-methylidenepropoxy)-3-fluoro-gamma-oxobenzenebutanoic acid

Following the procedure of Example 37, Steps B and C, but substituting an equivalent amount of the compound of Example 12, Step A, (for the compound of Example 1, Step B) in Step B of Example 37, the title compound was obtained, m.p. 132-134°.

Analysis, calculated: C, 65.49; H, 5.94.
Observed: C, 65.48; H, 5.99.

## EXAMPLE 40

4-(3-(4-acetyl-3-hydroxy-2-propyl-phenoxy)propylthio)-gamma-oxobenzenebutyronitrile

A: Preparation of 4-methylthio-gamma-oxobenzenebutyronitrile

A solution of 4-methylthiobenzaldehyde (15.2 g, 100 mmole) in dry DMF (50 ml) was added over 10 minutes to a solution of sodium cyanide (2.45 g, 50 mmole) in DMF (50 ml) at 35°C. After 5 minutes, acrylonitrile (4.0 g, 75 mmole) in DMF (100 ml) was added dropwise over 20 minutes. The reaction mixture stirred at 35° for 3 hours. The reaction mixture was cooled to room temperature, diluted with water (400 ml), extracted with chloroform, washed with 0.01N $H_2SO_4$, 5% $NaHCO_3$ and water, dried, and evaporated to a solid which was crystallized from ethyl acetate and hexane to yield the title compound, m.p. 122-124°.

B: Preparation of 4-methylthio-gamma-oxobenzenebutyronitrile-S-oxide

The compound of Step A above (205 mg, 1.0 mmole) was dissolved in chloroform (1 ml) and cooled to 0°. m-Chloroperbenzoic acid (200 mg, 1.1 mmole)

in chloroform (1 ml) was added to the cooled solution, and the reaction mixture warmed to room temperature over 30 minutes. Calcium hydroxide (210 mg) was added and the reaction stirred 15 minutes. The solution was filtered and evaporated to dryness to yield the title compound which was used directly in the following step.

C: Preparation of 4-trifluoroacetyloxymethylthio-gamma-oxo-benzenebutyronitrile

The compound of Step B above, (300 mg) was dissolved in 2.0 ml trifluoroacetic anhydride. The reaction mixture was heated at reflux for 30 minutes, cooled to room temperature, and evaporated to dryness. The title compound crystallized on standing. Mass spectrum m/e 317 ($M^+$).

D: Preparation of 4-mercapto-gamma-oxobenzene-butyronitrile

The compound of Step C above, (75 mg, 0.24 mmole) was dissolved in methanol (5 ml). To this solution was added in $Na_2CO_3$ (0.5 ml, 0.5 mmole) and the mixture was stirred at room temperature for 30 minutes. 6N HCl (0.2 ml) was added and the reaction mixture was extracted with chloroform, washed with brine, dried and evaporated to dryness to yield the title compound. Mass spectrum m/e 191 ($M^+$).

E: 4-(3-(4-acetyl-3-hydroxy-2-propyl-phenoxy) propylthio)-gamma-oxobenzenebutyronitrile

The compound of Step D above, (2.4 g, mmole), 2-hydroxy-3-propyl-4-(3-bromopropyloxy)-acetophenone (2.92 g) and $K_2CO_3$ (4.0 g) were

dissolved in methylethyl ketone (50 ml). The reaction mixture was heated at reflux for 90 minutes. The reaction mixture was poured into water, extracted with chloroform, washed with brine, dried and evaporated to yield the title compound. Recrystallization from ether:hexane (1:1) afforded the title compound as needles, m.p., 94-95°.
Analysis, calculated: C, 67.74; H, 6.39; S, 3.29.
Observed: C, 67.78; H, 6.65; s, 3.25.

EXAMPLE 41

5-(3(4-(3-(4-acetyl-3-hydroxy-2-propylphenoxy) propylthio)phenyl)-3-oxopropyl)-1H-tetrazole

The nitrile from Example 40, Step E, (1.0 g, 2.35 mmole) and tri-n-butyltin azide (1.0 g, 3.0 mmole) in dry THF (20 ml) were stirred at 70° for 48 hours, and then heated at reflux for 24 hours. The reaction mixture was cooled to room temperature, diluted with ether (20 ml) and concentrated HCl was added. After 1 hour, the solution was filtered, the solid was washed with ether and dried to yield the title compound.
Analysis, calculated: C, 61.52; H, 6.02; N, 11.96; S, 6.84.
Observed: C, 61.60; H, 6.13; N, 11.91; S, 6.99.

EXAMPLE 42

4-(3-(4-acetyl-3-hydroxy-2-propylphenoxy)propylsulfonyl)-gamma-oxobenzenebutyronitrile

The nitrile from Example 40, Step E, (680 mg, 1.6 mmole) and m-chloroperbenzoic acid (85%, 760 mg, 3.75 mmole) were stirred at 0° for two hours in 20 ml of $CH_2Cl_2$. Calcium hydroxide (400 mg) was

added to the reaction mixture which was stirred for 1.5 hours. The reaction mixture was filtered and evaporated to dryness, affording the title compound, mp 143-144°.

Analysis, calculated: C, 63.00; H, 5.95; N, 3.06; S, 7.01.

Observed: C, 63.09; H, 5.95; N, 3.38; S, 7.02.

EXAMPLE 43

5-(3-(4-(3-(4-acetyl-3-hydroxy-2-propylphenoxy) propylsulfonylphenyl)-3-oxopropyl)-1H-tetrazole

Following the procedure of Example 41, but substituting an equivalent amount of the nitrile from Example 42 for the nitrile from Example 40, Step E, there was obtained the title compound; m.p. 175-177°.

Analysis, calculated: C, 57.59; H, 5.64; N, 11.19; S, 6.41.

Observed: C, 57.64; H, 5.74; N, 11.18; S, 6.61.

EXAMPLE 44

4-(3-(4-acetyl-3-hydroxy-2-propyl-phenoxy)propylsulfonyl)-epsilon-oxobenzenehexanoic acid

A: Preparation of methyl 4-(3-bromopropylthio)-epsilon-oxobenzenehexanoate

Adipoylchloride monomethylester (5.95 g, 33.3 mmole) in dichloroethane (15 ml) was added dropwise to a suspension of aluminum chloride (4.5 g, 33.7 mmole) in dichloroethane (50 ml). The reaction mixture was stirred at room temperature for 20 minutes. 3-Bromopropylthiobenzene (7.0 g, 30.0 mmole) in dichloroethane (15 ml) was added dropwise to the stirred suspension over 30 minutes. Additional aluminum chloride (4.0 g ) was added to

complete the reaction. After 30 minutes the reaction mixture was poured into ice water (500 ml) and extracted with chloroform. The organic layer was washed with brine, dried and concentrated. The residue was crystallized from ether hexanes to yield the title compound.

NMR ($CDCl_3$) :1.73 (4H, m), 2.26 (4H, m), 2.93 (2H, m), 3.15 (2H, m), 3.50 (2H, m), 3.63 (3H, s), 7.32 (2H, d), 7.90 (2H, d).

B: Preparation of methyl 4-(3-(4-acetyl-3-hydroxy-2-propylphenoxy)-propylthio)-epsilon-oxo-benzenehexanoate

The compound of Step A above, (2.0 g, 5.36 mmoles), 2,4-dihydroxy-3-propylacetophenone (2.08 g, 10.72 mmole) and potassium carbonate (3.0 g, 21.44 mmole) were stirred in methyl ethyl ketone (100 ml) and heated at reflux for three hours. The reaction mixture was filtered and concentrated *in vacuo*. The residue was dissolved in ether-methanol and extracted with 1N NaOH (3 X 50 ml). The organics were dried and concentrated *in vacuo*. The residue was triturated with methanol, filtered and dried, to yield the title compound. NMR ($CDCl_3$): 0.94 (3H, m), 1.73 (6H, m), 2.28 (6H, m), 2.95 (2H, m), 3.22 (2H, m), 3.66 (3H, s), 4.17 (2H, m), 6.42 (1H, d), 7.40 (2M, d), 7.61 (1H, d), 7.90 (2H, d), 12.73 (1H, s).

C: Preparation of methyl 4-(3-(4-acetyl-3-hydroxy-2-propylphenoxy)-propylsulfonyl)-epsilon-oxo-benzenehexanoate

The compound of Step B above, (1.0 g, 2.05 mmoles) was dissolved in dry methylene chloride (25 ml) to which was added one equivalent of m-chloroperbenzoic acid in methylene chloride (10 ml). After 1 hour stirring at room temperature, additional m-CPBA (450 mg) was added. The reaction was complete in two hours. Calcium hydroxide (1.0 g) was added and the reaction mixture was stirred for 10 minutes. The mixture was filtered and the filtrate concentrated. The residue was purified by chromatography to yield the title compound. NMR ($CDCl_3$): 0.90 (3H, m), 1.50 (2H, m), 1.74 (4H, m), 2.33 (6H, m), 2.55 (3H, s), 3.03 (2H, m), 3.37 (2H, m), 3.68 (3H, s), 4.12 (2H, m), 6.39 (2H, d), 7.60 (2H, d), 8.12 (4H, m), 12.73 (1H, s).

D: Preparation of 4-(3-(4-acetyl-3-hydroxy-2-propylphenoxy)propylsulfonyl)-epsilon-oxobenzenehexanoic acid

The compound of Step C above, (800 mg, 1.54 mmoles, 1N NaOH (3.2 ml), water (3.2 ml) and THF (10 ml) were stirred at room temperature for three hours. The THF layer was removed and the aqueous portion diluted with water and extracted with ether. The aqueous portion was acidified with concentrated HCl. A solid precipitate formed which was extracted into ether, dried and concentrated. Trituration of the residue with ether-methanol afforded the title compound; mp 138-140°.

:

EXAMPLE 45

4(3-(4-acetyl-3-hydroxy-2-propylphenoxy)propylthio-) epsilon-oxobenzenehexanoic acid

The compound of Example 44, Step B (1.0 g, 2.055 mmole) was dissolved in THF (10 ml). To this solution was added 1N NaOH (4.1 ml). The reaction mixture was stirred at room temperature for two hours. Additional 1N NaOH was added and stirring continued for two hours. The THF solution was removed and the aqueous portion diluted with water and extracted with ether. The aqueous layer was acidified with concentrated HCl, extracted with chloroform, dried and evaporated. Trituration of the residue with ether/methanol afforded the title compound; m.p. 130-132°.

EXAMPLE 46

4-(3-(4-acetyl-3-hydroxy-2-propylphenoxy)-propylthio)-delta-oxo-benzenebutanol

A: Preparation of 4-(3-bromopropylthio)-gamma-oxobenzenebutanoic acid

A mixture of the product of Example 1, Step E (5 g), 1,3-dibromopropane (20 g) and $K_2CO_3$ (10 g) was refluxed in methylethylketone (100 ml) for 20 hours. The cooled reaction mixture was filtered, the solvent evaporated and the residue purified by chromatography over silica gel. The resulting ester of the title compound was hydrolyzed by dissolving in a mixture of 25 ml of methanol and 15 ml of 1N NaOH and stirring at 25°C for 4 hours. The reaction mixture was diluted with 100 ml of water, acidified with citric acid and extracted with 2 X 100 ml of EtOAc. The combined organic layers were washed with

brine, dried over $Na_2SO_4$ and evaporated to yield the title compound which was sufficiently pure for use in the next step, m.p. 91-94°.

B: Preparation of 4(3-bromopropylthio)-delta-oxo-benzenebutanol (A) and 4-(3-bromopropylthio)delta-hydroxy-benzenebutanol (B)

4-(3-Bromopropylthio)-gamma-oxobenzenebutanoic acid (3.4 g, 10.3 mmoles) was dissolved in THF (25 ml) and cooled to -15°. Borane-THF complex (10 ml, 10.4 mmoles) was added dropwise over one hour. The solution warmed to room temperature with stirring over 12 hours. The reaction mixture was diluted with methanol, stirred for 30 minutes and evaporated *in vacuo*. The residue was treated with an excess of diazomethane and following workup and separation by HPLC the title compounds were obtained.

C: Preparation of 4-(3-(4-acetyl-3-hydroxy-2-propylphenoxy)-propylthio)-delta-oxo-benzenebutanol

The compound A of Step A above (96 mg, 0.29 mmoles), 2,4-dihydroxy-3-propylacetophenone (78 mg, 0.4 mmole) and potassium carbonate (270 mg, 2.0 mmoles) were refluxed in methyl ethyl ketone (3 ml) for twelve hours. The reaction mixture was filtered and purified by chromatography to yield the title compound; m.p. 73-75°.

Analysis, calculated: C, 66.95; H, 7.02; S, 7.45.
Observed: C, 67.00; H, 7.11; S, 7.22.

EXAMPLE 47

4(3-(4-acetyl-3-hydroxy-2-propylphenoxy)propylsulfonyl)-delta-oxobenzenebutanol

The compound of Example 46, Step B (330 mg, 0.767 mmoles) was dissolved in chloroform and cooled to 0°. m-Chloroperbenzoic acid (328 mg, 1.57 mmoles) was added and the reaction stirred for one hour. Calcium hydroxide (195 mg) was added and the reaction stirred for 90 minutes at room temperature. The mixture was filtered and the filtrate evaporated to dryness. The residue crystallized from ethyl acetate-hexane to yield the title compound; m.p. 99-100°. Analysis, calculated: C, 62.32; H, 6.54; S, 6.93. Observed: C, 62.23; H, 6.50; S, 6.68.

EXAMPLE 48

4-(3-(4-acetyl-3-hydroxy-2-propylphenoxy)propylthio-beta,epsilon-dioxobenzenepentanol

A: Preparation of 4-(3-(4-acetyl-3-hydroxy-3-propyl-phenoxy)propylthio-alpha-isobutylyloxycarbonyloxy-alpha,delta-dioxobenzenebutane

Isobutylchloroformate (260 µl, 2.0 mmoles) was added to 10 ml methylene chloride and cooled to -30°. The compound of Example 6, (890 mg, 2.0 mmoles) was dissolved in a mixture of methylene chloride (6 ml) and triethylamine (280 µl) and added dropwise to the -30° solution. The reaction mixture was maintained at -30° for 30 minutes, then held at 0° for 30 minutes. The reaction mixture was concentrated *in vacuo* to yield the title compound which was used without further purification in the next step.

B: Preparation of 4-(3-(4-acetyl-3-hydroxy-2-propylphenoxy)propylthio-alpha-diazomethyl-alpha,gamma-dioxobenzenebutane

The crude compound from Step A above (1.1 g) was dissolved in toluene (20 ml) and cooled to 0°. An excess amount of diazomethane was added to the solution and the reaction mixture was stirred for twelve hours. The reaction mixture was purged with air, filtered and concentrated *in vacuo*, to yield the title compound which was used in the next step without further purification.

C: Preparation of 4-(3-(4-acetyl-3-hydroxy-2-propylphenoxy)propylthio-beta-epsilon-dioxobenzenepentanol

The crude compound from Step B above (800 mg) was dissolved in chloroform (20 ml) to which was added trifluoroacetic acid (excess). The reaction mixture stirred at room temperature for two hours, was filtered, and concentrated *in vacuo*. The residue was purified by column chromatography to yield the title compound, m.p. 87-89°.

## EXAMPLE 49

4-(3-(4-acetyl-3-acetoxy-2-propylphenoxy)propylthio)-gamma-oxo-benzenebutanoic acid

To a suspension of the compound of Example 6, (1.2 g, 2.7 mmoles) and dihydropyran (0.8 ml, 4.4 mmoles) in methylene chloride (20 ml) was added p-toluenesulfonic acid monc ydrate (5 mg). The reaction mixture was stirred at room temperature for two hours. The solution was cooled to 0° and triethylamine (6 ml), 4-dimethylaminopyridine (DMAP)

(15 mg) and acetic anhydride (5 ml) were added. The resulting solution stirred at room temperature for two hours. The solution was washed with 5% $NaHCO_3$ (2X), water, 1N HCl and concentrated in vacuo. The residue was dissolved in THF (6 ml) and stirred with 1N HCl (1 ml) at room temperature for one hour. Dichloromethane (50 ml) and water (50 ml) were added to the solution and the organic layer was collected, washed with brine (4X) dried ($Na_2SO_4$) and concentrated in vacuo. The crystalline residue was washed with hexane and recrystallized to afford the title compound, m.p. 121-123°.
Analysis, calculated: C, 64.18; H, 6.21; S, 6.59.
Observed: C, 64.20; H, 6.30; S, 6.62.

EXAMPLE 50

4-(3-(4-acetyl-3-acetoxy-2-propylphenoxy)-propylsulfonyl)-gamma-oxobenzenebutanoic acid

Following the procedure of Example 49, but substituting an equivalent amount of the compound of Example 29, for the compound of Example 6, was obtained the title compound, m.p. 128-131°.
Analysis, calculated: C, 60.22; H, 5.83; S, 6.18.
Observed: C, 60.30; H, 5.95; S, 6.29.

EXAMPLE 51

4-(3-(4-acetyl-3-hydroxy-2-propylphenoxy)-propylsulfonyl-gamma-oxobenzenebutyramide

A solution of the compound of Example 29, (1 g, 2.12 moles) and triethylamine (310 µl, 2.2 mmoles) in methylene chloride (5 ml) was cooled to -30°. Isobutylchloroformate (286 µl, 2.2 mmoles) was added to the -30° solution over ten minutes. The

reaction mixture was warmed to 0° and stirred for 30 minutes. Anhydrous ammonia was bubbled into the cold solution for 10 minutes. The reaction mixture warmed to room temperature and stirred for 15 minutes. The solution was filtered and the solids washed with chloroform, to yield the title compound, m.p. 198-199° (dec.).

Analysis, calculated: C, 60.62; H, 6.15; N, 2.94; S, 6.74.

Observed: C, 60.53; H, 6.32; N, 2.98; S, 6.84.

EXAMPLE 52

N-methylsulfonyl 4-(3-(4-acetyl-3-hydroxy-2-propylphenoxy)propylthio-gamma-oxobenzenebutyramide

A solution of the compound of Example 6, (1.32 g, 3.0 mmoles) and triethylamine (0.42 ml, 3.0 mmoles) in methylene chloride (6 ml) was added to a solution of isobutylchloroformate (0.39 ml, 3.0 mmoles) in methylene chloride (6 ml) at -30°. The mixture was stirred at 0° for thirty minutes, then triethylamine (1.26 ml, 9.7 mmoles) and methanesulfonamide (0.60 g, 9.5 mmoles) were added and the reaction stirred at room temperature for 24 hours. The mixture was poured into water, acidified with 1N HCl, and extracted with methylene chloride (3X). The combined organic extracts were washed with brine, dried ($Na_2SO_4$) and concentrated *in vacuo*. The residue was washed with ether and recrystallized to afford the title compound as needles, m.p. 148-150°.

Analysis, calculated: C, 57.56; H, 5.99; N, 2.69; S, 12.29.

Observed: C, 57.89; H, 6.31; N, 2.90; S, 12.29.

## EXAMPLE 53

5(4-(3-(4-acetyl-3-hydroxy-2-propylphenoxy)propylsulfonyl)phenyl)-2(3H)-furanone

The compound of Example 29, (2.0 g, 4.197 mmoles) was suspended in chloroform (120 mls) to which was added 1,1-dichloromethylmethyl ether (10 ml). The reaction mixture was heated at 40° for 48 hours, and concentrated *in vacuo*. The residue was recrystallized to afford the title compound, m.p. 135-136°.

Analysis, calculated: C, 62.87; H, 5.72; S, 6.99.
Observed: C, 62.70; H, 5.75; S, 7.11.

## PRODRUG EXAMPLES

The following Table lists various prodrug derivatives (Formula XV) of the compounds of the present invention. The processes for preparing these compounds are discussed *supra*.

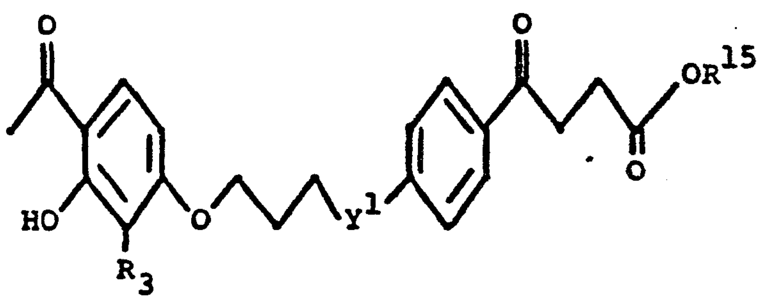

XV

$R_3$ in Formula XV is n-propyl.

TABLE I

| EXAMPLE NO. | L-Number | $Y^1$ | $OR^{15}$ |
|---|---|---|---|
| 54 | L-649,874-00M01 | S | 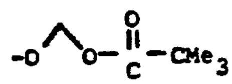 |
| 55 | L-649,888-00P01 | $SO_2$ | 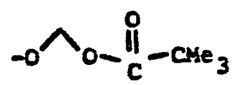 |
| 56 | - | S | 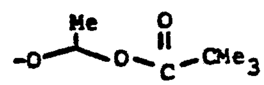 |
| 57 | L-649,948-00E01 | $SO_2$ | 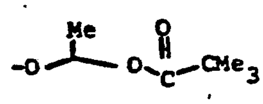 |
| 58 | L-649,875-00W01 | S | 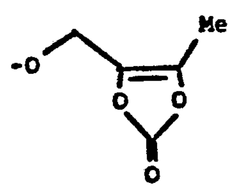 |
| 59 | L-649,893-00N01 | $SO_2$ | 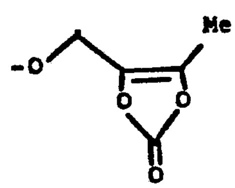 |
| 60 | L-649,876-00E01 | S | 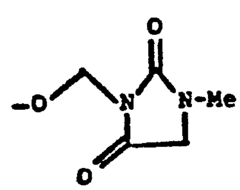 |

TABLE I (cont'd)

| | | | |
|---|---|---|---|
| 61 | L-649,877-00N01 | $SO_2$ | 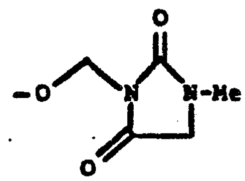 |
| 62 | - | S | 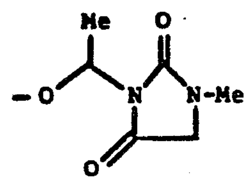 |
| 63 | L-649,951-00L01 | $SO_2$ | 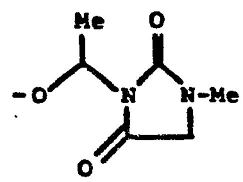 |
| 64 | L-649,930-00T01 | S | 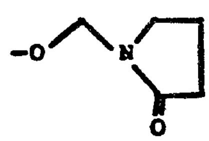 |
| 65 | L-649,931-00B01 | $SO_2$ | 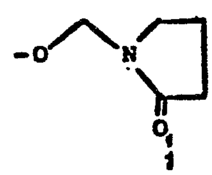 |
| 66 | L-649,932-00K01 | S | 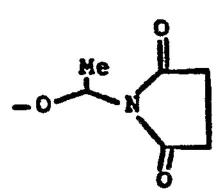 |

TABLE I (cont'd)

| | | | |
|---|---|---|---|
| 67 | L-649,933-00001 | $SO_2$ | 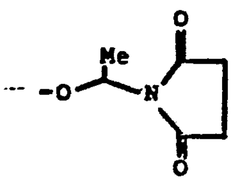 |
| 68 | - | S | 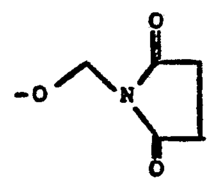 |
| 69 | L-649,952-00V01 | $SO_2$ | 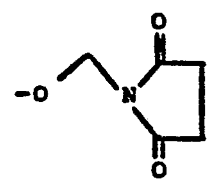 |

EXAMPLE 70

4(3(4-Acetyl-3-hydroxy-2-propylphenoxy)propyl-oxy)-gamma-oxo-beta,beta-dimethylbenzenebutanoic acid

A: Preparation of Methyl 4-methoxy-gamma-oxo-beta-methylbenzenebutanoate

A solution of diisopropylamine (5.1 ml) in anhydrous THF (90 ml) at 0°C is treated under $N_2$ atmosphere with n-butyllithium (1.6 M, 22 ml) for 20 minutes. The mixture was cooled to -78°C, and a solution of 1(4-methoxyphenyl)-1-oxopropane (5 g) in anhydrous THF (40 ml) is added. The mixture was stirred 30 minutes at -78°C, then methyl bromoacetate (3.05 ml) was added dropwise and then mixture was warmed to ambient temperature over 2 hours. After stirring 18 hours at room temperature the mixture was poured onto ice, water and conc. HCl (3 ml) was added and the solution was extracted with dichloromethane. The organic extracts were washed with brine, dried over $Na_2SO_4$ and concentrated to an oil which was purified by chromatography on silica gel to provide the title compound as an oil.

NMR ($CDCl_3$): 1.2 (3H, m), 2.2-3.1 (2H, m), 3.6 (3H, s), 3.85 (3H, 5), 3.7-4.0 (1H, m), 6.9 (2H, d), 8.0 (2H, d).

B: Preparation of Methyl 4-methoxy-gamma-oxo-beta, beta-dimethylbenzenebutanoate

The product from Step A, (3.05 g) was stirred in methanol (30 ml) and in NaOH (15 ml) at ambient temperature for 1 hour. The mixture was diluted with brine (50 ml), washed with ethyl acetate, acidified with conc. HCl and extracted with ethyl acetate (2 x 50 ml). The second organic

extract was washed with brine, dried ($Na_2SO_4$) and reduced to dryness. The resulting oil was dissolved in anhydrous THF (20 ml) and added to a suspension of potassium hydride (1.56 g) in THF (20 ml) under nitrogen at -40°C. DMSO (50 µl) was added and the mixture was warmed to 0°C for 1/2 hr., cooled to -40° and then treated with methyl iodide (3.7 ml). After 1/2 hour at -40°C, the mixture was stirred at ambient temperature for 18 hours, then poured onto an excess of ice-conc. HCl mixture and was extracted with ethyl acetate. The organic extract was washed with brine, dried ($Na_2SO_4$) and reduced to dryness. The resulting oil was dissolved in anhydrous methanol, saturated with HCl gas, and after 1 hour reduced to dryness to yield an oil which was purified by chromatography on silica gel to yield the title compound as an oil. NMR ($CDCl_3$): 1.46 (6H, s), 2.78 (2H, s), 3.60 (3H, s), 3.82 (3H, s), 6.88 (2H, d), 7.80 (2H, d).

C: Preparation of Methyl 4-hydroxy-gamma-oxo-beta, <u>beta-dimethylbenzenebutanoate</u>

The product from Step B, (1.72 g) in $CH_2Cl_2$ (10 ml) at -78°C, was treated with boron tribromide (22 ml of a 1M solution in $CH_2Cl_2$). After 1/2 hour at -78°C the mixture was stirred at ambient temperature for 18 hours. The mixture was cooled to 0°C treated with methanol (5 ml) then washed with water, brine, dried over $Na_2SO_4$ and reduced to dryness to yield an oil which was purified by chromatography on silica gel to provide the title compound as an oil.

NMR ($CDCl_3$): 1.45 (6H, s), 2.78 (2H, s), 3.60 (3H, s), 6.80 (2H, d), 7.67 (2H, d).

D. Preparation of Methyl 4-(3-(4-acetyl-3-hydroxy-2-propylphenoxy)propyloxy)-gamma-oxo-beta,beta-dimethylbenzenebutanoate

A mixture of the product from Step C (250 mg), 4-(3-bromopropyl)-2-hydroxy-3-propylacetophenone (315 mg), and potassium carbonate (410 mg) in methyl ethyl ketone (10 ml) was refluxed under $N_2$ for 4 hours then stirred at ambient temperature for 18 hours. The mixture was filtered, washed with brine, evaporated to dryness and the resulting oil was purified by chromatography on silica gel to provide the title compound as an oil. NMR ($CDCl_3$) 0.90 (3H, t), 1.43 (6H, s), 1.52 (2H, m), 2.30 (2H, m), 2.50 (3H, s), 2.55 (2H, m), 2.75 (2H, s), 3.57 (3H, s), 4.18 (4H, m), 6.41 (1H, d), 6.88 (2H, d), 7.53 (1H, d), 7.72 (2H, d).

E. Preparation of 4-(3-(4-acetyl-3-hydroxy-2-propylphenoxy)propyloxy)-gamma-oxo-beta,beta-dimethylbenzebutanoic acid

The ester from Step 4 (740 mg) in methanol (1 ml) and THF (10 ml) was treated with 1N NaOH (4 ml) at ambient temperature for 2 hours. The mixture was acidified with conc. HCl, diluted with brine, extracted with ethyl acetate and the organic extracts were washed with brine, dried ($Na_2SO_4$) and evaporated to dryness to yield an oil which was purified by chromatography on silica gel to yield the title compound as a viscous oil.
NMR: ($CDCl_3$): 0.89 (3H, t), 1.40 (8H, s and m), 2.30 (2H, m), 2.50 (3H, s), 2.5-2.8 (4H, m), 4.22 (4H, m), 6.47 (1H, d), 6.90 (2H, d), 7.49 (3H, m), 12.70 (1H, s).

## EXAMPLE 71

**D,L-Sodium 4-(3-(4-acetyl-3-hydroxy-2-propylphenoxy) propyloxy)-gamma-hydroxy-beta,beta-dimethylbenzenebutanoate**

A: Preparation of 4-hydroxy-gamma-hydroxy-beta,beta-dimethylbenzenebutanoic acid gamma lactone

The phenol from Example 70, Step C, (1.19 g) in dioxane (5 ml) was treated with sodium borohydride (190 mg) and the mixture was stirred 18 hours at ambient temperature. The solution was poured into 1N HCl (10 ml) and extracted with ethyl acetate. The extracts were washed with brine, dried ($Na_2SO_4$) and concentrated to dryness. The residue was recrystallized from chloroform-hexane to provide the title compound, m.p. 157-158°C.

B: Preparation of 4-(3-(4-acetyl-3-hydroxy-2-propyl-phenoxy)-propoxy)-gamma-hydroxy-beta,beta-dimethyl-benzenebutanoic acid gamma lactone

The lactone from Step A (85 mg), 4-(3-bromo-propyloxy)-3-propyl-2-hydroxyacetophenone (160 mg) and potassium carbonate (210 mg) were refluxed together in methyl ethyl ketone (5 ml) under $N_2$ atmosphere for 8 hours. The mixture was cooled, diluted with ethyl acetate, washed with brine, dried ($Na_2SO_4$) and evaporated to an oil which was purified by chromatography on silica gel to provide the title compound as an oil, NMR ($CDCl_3$): 0.68 (3H, s), 0.90 (3H, t), 1.22 (3H, s), 1.52 (2H, m), 2.30 (2H, m), 2.47 (2H, m), 2.53 (3H, s), 2.60 (2H, m), 4.18 (4H, m), 5.08 (1H, s), 6.47 (1H, d), 6.87 (2H, d), 7.18 (2H, d), 7.60 (1H, d), 12.72 (1H, s).

C. Preparation of D,L-sodium 4-(3-(4-acetyl-3-hydroxy-2-propylphenoxy)propyloxy) ;amma-hydroxy-beta,beta-dimethylbenzenebutanoate

The product from Step B (950 mg) was stirred in methanol (2 ml), THF (20 ml) and 1N NaOH (4.5 ml) for 2.5 hours. The mixture was concentrated to near dryness, taken up in water (10 ml) and applied to a column of XAD-8 resin. After standing 1 hour the columnd was washed with water until the effluent pH was neutral. Methanol (50 ml) was applied to the column and the effluent was concentrated to dryness and the residue was triturated with ether to provide the title compound as a powder, mp 101-103°.

Analysis, calculated: $C_{26}H_{33}O_7N_2$: C, 64.99; H, 6.92.

Observed: C, 65.18; H, 7.04.

EXAMPLE 72

4-(3-(4-acetyl-3-hydroxy-2-propylphenoxy)propylthio)-gamma-oxo-beta,beta-dimethylbenzenebutanoic acid

A: Preparation of 1(4-methylthiophenyl)-1-propanone

To a mixture of thioanisole (5.0 g) and propionyl chloride (3.9 ml) at 0°C in dichloroethane (80 ml) was added $AlCl_3$ (6.4 g) in portions. The mixture was stirred 12 hours at ambient temperature, poured into water (200 ml) and conc. HCl (2 ml), then extracted with $CH_2Cl_2$. The extract was reduced to dryness to provide the title compound mp 60-61°C after purification by chromatography on silica gel.

B. Preparation of methyl 4-methylthio-gamma-oxo-beta-methylbenzenebutyrate

The ketone from Step A (180 mg) in THF (2 ml) was added to a solution of lithium diisopropylamide (1.1 mmole) in THF (3 ml) at -78° under $N_2$ atmosphere. After 15 minutes methyl bromoacetate (100 µl) was added and the mixture was warmed to ambient temperature and stirred 12 hours. The mixture was poured into water (15 ml) and conc. HCl (2 ml), extracted with $CH_2Cl_2$ and the extracts were dried ($Na_2SO_4$) evaporated to dryness and the residue purified by chromatography on silica gel to provide the title compound as an oil. NMR ($CDCl_3$): 1.2 (3H, d), 2.55 (3H, s), 2.3-3.1 (2H, m), 3.65 (3H, s), 3.95 (1H, m), 7.3 (2H, d), 7.95 (2H, d).

C. Preparation of 4-methylthio-gamma-oxo-beta-methylbenzenebutanoic acid

The ester from Step B (1.07 g) was saponified with 1N NaOH (5 ml) and methanol (5 ml) and THF (5 ml) to provide after acidification, extraction into $CH_2Cl_2$ and evaporation to dryness the title compound, mp 69-71°C.

D. Preparation of Methyl 4-methylthio-gamma-oxo-beta, beta-dimethylbenzenebutyrate

The acid from Step C (3.03 g) in THF (10 ml) was added to a suspension of KH (1.9 g) in THF (50 ml) at -40° under argon. DMSO (50 µl) was added, the mixture was stirred at -5°C until gas evolution ceased, then recooled to -40°C followed by addition of methyl iodide (1.8 ml). After 0.5 hour at -40° and 12 hours at ambient temperature, the mixture was diluted with water, acidified with HCl and extracted with $CH_2Cl_2$. The organic extracts were dried

($Na_2SO_4$) and evaporated to an oil which was treated with excess diazomethane in ether. After concentration the residue was purified by chromatography on silica gel to provide the title compound.
NMR ($CDCl_3$): 1.40 (6H, s), (2H, m), 2.55 (3H, s), 2.75 (2H, s), 3.67 (3H, s), 7.23 (2H, d), 7.57 (2H, d).

E. Preparation of methyl 4-(3-(4-acetyl-3-hydroxy-2-propylphenoxy)propylthio-gamma-oxo-beta,beta-dimethylbenzenebutyrate

The ester from Step D (0.71 g) was treated in $CH_2Cl_2$ (10 ml) at 0°C with m-chloroperbenzoic acid (0.57 g) for 10 minutes, $Ca(OH)_2$ in excess was added and after 10 minutes at ambient temperature the mixture was filtered and evaporated to dryness. The residue was refluxed with trifluoroacetic anhydride (7 ml) for 20 minutes then concentrated to dryness. The residue was mixed with 2-hydroxy-3-propyl-4-(3-bromopropyloxy)acetophenone (0.945 g) and $K_2CO_3$ (1.2 g) in methyl ethyl ketone (10 ml) and water (50 µl) and the mixture was stirred at 20°C for 15 hours. The mixture was concentrated, partitioned between dilute HCl and $CH_2Cl_2$. The organic extracts were concentrated and purified by chromatography on silica gel to provide the title compound. NMR ($CDCl_3$) 1.40 (6H, s), 2.77 (2H, s), 7.13 (2H, d), 7.37 (2H, d), 12.75 (1H, s).

F: Preparation of 4-(3-(4-acetyl-3-hydroxy-2-propylphenoxy)propylthio)-gamma-oxo-beta,beta-dimethylbenzenebutanoic acid

The ester from Step E (0.984 g) was saponified, acidified and the product purified by chromatography on silica gel to provide the title compound.

Analysis, calculated: C, 66.07; H, 6.83; S, 6.79.
Observed: C, 66.14; H, 6.86; S, 6.51.

EXAMPLE 73

Sodium 4-(3-(4-acetyl-3-hydroxy-2-propylphenoxy)propylthio)-gamma-hydroxy-beta,beta-dimethylbenzenebutyrate

A: Preparation of methyl 4(3-bromopropylthio)-gamma-oxo-beta,beta-dimethylbenzenebutyrate

Following the procedure described in Example 72, Step E but substituting three equivalents of dibromopropane in place of 4(3-bromopropyloxy) 2-hydroxy-3-propyl acetophenone was obtained the title compound as an oil. NMR ($CDCl_3$): 1.40 (6H, s), 2.0-2.3 (2H, m), 2.77 (2H, s), 3.13 (2H, t), 3.50 (2H, t), 3.67 (3H, s), 7.23 (2H, d), 7.57 (2H, d).

B: Preparation of 4-(3-bromopropylthio)-gamma-hydroxy-beta,beta-dimethylbenzenebutanoic acid gamma lactone

The ester from Step A (1.4 g) in methanol (10 ml) was treated at -15°C with ceric chloride (2 mg) and $NaBH_4$ (100 mg) for 1 hour. The mixture was diluted with water, (100 ml) acidified with 1N HCl and extracted with $CH_2Cl_2$. The extracts were left for 2 days at room temperature, concentrated and purified by chromatography on silica gel to provide the title compound as an oil. NMR ($CDCl_3$): 0.20 (3H, s), 1.27 (3H, s), 2.0-2.3 (2H, m), 2.37 (1H, d), 2.60 (1H, d), 3.07 (2H, t), 3.50 (2H, t), 5.10 (1H, s), 7.13 (2H, d), 7.33 (2H, d).

C: Preparation of 4-(3-(4-acetyl-3-hydroxy-2-propylphenoxy)propylthio-gamma-hydroxy-beta,beta-dimethylbenzenebutanoic acid gamma lactone

The lactone from Step B (0.95 g) and 2,4-dihydroxy-3-propylacetophenone (0.54 g) were placed with $K_2CO_3$ (1.2 g) in methyl ethyl ketone (10 ml) for 4 hours. The mixture was filtered, diluted with $CH_2Cl_2$, washed with water, dried ($Na_2SO_4$) concentrated and chromatographed on silica gel to provide the title compound as an oil. Mass spectrum m/e 456 ($M^+$).

D. Preparation of Sodium 4-(3-(4-acetyl-3-hydroxy-2-propylphenoxy)propylthio-gamma-hydroxy-beta, beta-dimethylbenzenebutyrate

The lactone from Step C (0.936 g) was saponified with 1N NaOH (4 ml) THF (4 ml) and MeOH (4 ml) for 45 minutes at ambient temperature. The mixture was concentrated to near dryness and then purified on XAD-8 resin to provide the title compound as a foam.

Analysis, calculated: C, 62.88; H, 6..70; S, 6.46.
Observed: C, 62.71; H, 6.81; S, 6.46.

EXAMPLE 74

Resolution of Sodium (beta S*, gamma R*)-4-(3-(4-acetyl-3-hydroxy-2-propylphenoxy)propylthio-gamma-hydroxy-beta-methylbenzenebutanoate into the (+) isomer and the (-)-isomer

A: Preparation of methyl (beta S, gamma R)-4-(3-(4-acetyl-3-hydroxy-2-propylphenoxy)propylthio)-gamma-(d-(alpha-methoxy)phenylacetoxy)-beta-methyl-benzenebutanoate and methyl (beta R, gamma S)-4(3-(4-acetyl-3-hydroxy-2-propylphenoxy)propylthio)-gamma-(d-(alpha-methoxy)phenylacetoxy)-beta-methyl-benzenebutanoate

To the lactone (360 mg) from Example 75 in THF (4 ml) and MeOH (1.5 ml) was added 1N NaOH (2 ml). After 30 minutes at room temperature the solution was evaporated and ethyl acetate (20 ml) was added. The mixture was cooled to 0° and acidified with HCl (1N). The ethyl acetate layer was separated and the aqueous layer was re-extracted with ethyl acetate. The combined organic layers were dried ($Na_2SO_4$) and evaporated to give crude hydroxy acid. To the crude acid in ethyl acetate was added excess $CH_2N_2$. The reaction mixture was stirred 10 minutes at room temperature and evaporated. To the residue was added THF (10 ml), $Et_3N$ (.6 ml), DCC (500 mg), DMAP (5 mg) and d-α-methoxy mandelic acid (500 mg). The reaction mixture was stirred at room temperature 2 hours and evaporated. The residue was passed through a short column of $SiO_2$ (~20 g) using 30% ethyl acetate in hexane as eluant. The combined product fractions were evaporated. Purification of the residue using HPLC using 20% ethyl acetate on Waters μ-porasil column afforded two isomers.

Less Polar Isomer: p.m.r. ($CDCl_3$): 0.7 (d, 3H), 1.0 (t, 3H), 1.6 (m, 2H), 1.8-2.2 (m, 4H), 2.6 (s, 3H), 2.7 (t, 2H), 3.1 (t, 2H), 3.3 (s, 3H), 3.5 (s, 3H), 4.1 (t, 2H), 4.7 (s, 1H), 5.6 (d, 1H), 6.3 (d, 1H), 7.0-7.4 (m, 9H), 7.6 (d, 1H), 12.8 (s, 1H).

More Polar Isomer: n.m.r. ($CDCl_3$): .9 (d, 3H), 1.5 (m, 2H), 2.0-2.5 (m, 4H), 2.6 (s, 3H), 2.65 (t, 2H), 3.1 (t, 2H), 3.4 (s, 3H), 3.65 (s, 3H), 4.1 (t, 2H), 4.7 (s, 1H), 5.6 (d, 1H), 6.3 (d, 1H), 7.0 (m, 2H), 7.4 (s, 5H), 7.6 (d, 1H), 12.8 (s, 1H).

B. Preparation of the (+)-isomer of Sodium (beta S*, gamma R*)-4-(3-(4-acetyl-3-hydroxy-2-propylphenoxy)propylthio-gamma-hydroxy-beta-methylbenzenebutanoate

A solution of least polar isomer from Step A (200 mg) in MeOH (5 ml), THF (5 ml) and 1N NaOH (1 ml) was stirred at room temperature. After 2 hours 1N NaOH (5 ml) was added. After 4 hours the reaction mixture was evaporated and redissolved in $H_2O$ (2 ml). The solution was applied to an XAD column (30 ml resin). The column was washed with $H_2O$ (200 ml) and the product was then eluted with MeOH (150 ml). Evaporation of the MeOH afforded the title compound as an oil.

$[\alpha]_D$ + 9.5° (c=2.0, MeOH).

C. Preparation of the (-)-isomer of Sodium (beta S*, gamma R*)-4-(3-(4-acetyl-3-hydroxy-2-propylphenoxy)propylthio-gamma-hydroxy-beta-methylbenzenebutanoate

When the same procedure as described for the least polar isomer was applied to the more polar isomer from Step A (155 mg) there was obtained the title compound as an oil.

$[\alpha]_D$ -9.2° (c=2.0, MeOH).

## EXAMPLE 75

**(Beta S*, gamma R*)-4-(3-(4-acetyl-3-hydroxy-2-propylphenoxy)propylthio)-gamma-hydroxy-beta-methylbenzenebutanoic acid gamma lactone**

A: Preparation of 4-methoxy-gamma-oxobenzenebutanoic Acid

Anisole (70.0 g) and succinic anhydride (65.0 g) were dissolved in 1,2-dichloroethane (1 liter) and the mixture was cooled to 0°C. To the resulting suspension there was added, in portions, $AlCl_3$ (172 g) and the resulting mixture was stirred with a mechanical stirrer for 1 hour. The mixture was then poured into a mixture of ice and water (about 1 liter) containing 50 ml of concentrated HCl. The resulting white solid was collected by filtration, washed with water and air dried to yield the title compound, mp 145-147°C.

B: Preparation of 4-hydroxy-gamma-oxobenzenebutanoic acid, methyl ester

A mixture of the compound from Step A (77.3 g), 48% HBr (310 ml), and acetic acid (620 ml) was heated under reflux for 18 hours. The resulting mixture was cooled to room temperature and poured into 3 liters of water. The resulting solution was extracted with ethyl acetate (3 x 500 ml). The combined organic layers were washed with water (4 x 200 ml), dried over $Na_2SO_4$, the solvents were removed by evaporation and the residue was dissolved in 10% HCl/methanol (500 ml). After 1 hour at room temperature the volatile components were removed by evaporation *in vacuo*. The resulting residue was triturated with hexane to yield the title compound, mp 115-116°.

C: Preparation of 4-dimethylthiocarbamoyloxy-gamma-oxobenzenebutanoic acid, methyl ester

A solution of the product from Step B, 25 g, in anhydrous dimethylformamide (DMF) (300 ml) was cooled to 0° and 99% NaH, 3.46 g, was added in two portions. The mixture was stirred for 1 hour at 0° then dimethylthiocarbamoyl chloride, 19.3 g, was added and the mixture heated at 90° under a $N_2$ atmosphere for 1.5 hours. The mixture was cooled to room temperature and diluted with water to 1,200 mL. The resulting solution was then extracted with ethyl acetate (3 x 50 ml). The combined organic layers were washed with brine and then dried over $Na_2SO_4$ and evaporated to dryness *in vacuo* to yield a residue which was purified by chromatography on silica gel to yield the title compound, mp 62-64°.

D: Preparation of 4-dimethylcarbamoylthio-gamma-oxobenzenebutanoic acid, methyl ester

The compound from Step C, 29.6 g, was heated at 200° for 10 hours under an $N_2$ atmosphere. The mixture was cooled to room temperature, dissolved in methylene chloride and purified by chromatography on silica gel to provide the title compound, mp 98-100°.

E: Preparation of methyl 4-dimethylcarbamoyl-thio-gamma-oxo-beta-methylbenzenebutanoate

The compound from Step D, (10.0 g, 33.9 mmoles) was dissolved in THF (100 ml) and added dropwise to a stirred mixture of potassium hydride (1.6 g, 40.7 mmoles) in THF (10 ml) at -45°C. The reaction mixture was warmed to 0° to initiate the reaction, then cooled to -45°C. After 30 minutes,

the reaction mixture was warmed to 0°C, and stirred for 10 minutes. A solution of methyl iodide (3.17 ml, 50.9 mmoles) in THF (10 ml) was added slowly to the cold solution. The mixture was stirred at 0° for one hour. The reaction mixture was diluted with ether, washed with water, brine, dried and evaporated to yield an oil which was purified by HPLC to yield the title compound.

Analysis, calculated: C, 58.23; H, 6.19; N, 4.53; S, 10.36.

Observed: C, 58.43; H, 6.31; N, 4.76; S, 10.39.

F: Preparation of (beta S*, gamma R*) 4-dimethylcarbamoylthio-gamma-hydroxy-beta-methylbenzenebutanoic acid gamma lactone (cis isomer) and (beta R*, gamma R*) 4-dimethylcarbamoylthio-gamma-hydroxy-beta-methylbenzenebutanoic acid gamma lactone (trans isomer)

To a solution of the compound from Step E above (10.4 g, 33.7 mmoles) in methanol (150 ml) was added cesium chloride monohydrate (50 mg). The solution was cooled to -20° and sodium borohydride (640 mg, 16.8 mmoles) was added in portions. The reaction mixture stirred at -20° for two hours. 2N NaOH (50 ml) was added and the mixture stirred at room temperature for one hour. The mixture was diluted with water (500 ml), cooled to 0°, acidified with concentrated HCl and extracted with ethyl acetate (3X). The combined organic extracts were washed with brine, dried and concentrated to an oil. The oil was dissolved in methylene chloride (150 ml) and trifluoroacetic acid (1 ml) was added. The reaction mixture stirred at room temperature for one

hour. The reaction mixture was evaporated to an oil which crystallized on standing. The solid was purified by HPLC to afford pure cis (mp 116-118°) and pure trans (m.p. 74-76°) isomers of the title compound.

G: Preparation of (beta S*, gamma R*)-4-(3-(4-acetyl-3-hydroxy-2-propylphenoxy)propylthio)-gamma-hydroxy-beta-methylbenzenebutanoic acid gamma lactone

The cis-isomer of the compound of Step F above (4.50 g, 16.1 mmoles) was suspended in methanol (160 ml). To the suspension was added 2N NaOH (80 ml, 160 mmoles) and the mixture was heated at reflux for five hours. The mixture was cooled to room temperature and 4-(3-bromopropoxy)-3-propyl-2-hydroxyacetophenone (7.61 g, 24.2 mmoles) was added. The mixture stirred at room temperature for three hours, and was then heated at reflux for two hours. The reaction mixture was cooled to room temperature, diluted with water (500 ml), acidified with concentrated HCl and extracted with ethyl acetate (2X). The organic extracts were washed with brine, dried and concentrated to an oil. The oil was dissolved in methylene chloride (100 ml) and trifluoroacetic acid (1 ml) was added. The mixture stirred at room temperature for one hour. The solution was evaporated to an oil which was purified by HPLC to yield the title compound as a white solid, m.p. 92-94°.

Analysis, calculated: C, 67.84; H, 6.83; S, 7.25.
Observed: C, 67.75; H, 6.84; S, 7.47.

EXAMPLE 76

(Beta S*, gamma R*) 4-(3-(4-acetyl-3-hydroxy-2-propylphenoxy)propylsulfonyl)-gamma-hydroxy-beta-methylbenzenebutanoic acid gamma lactone

The compound of Example 75, Step G (550 mg, 1.24 mmoles) was dissolved in methylene chloride (5 ml) and a solution of m-chloroperbenzoic acid (606 mg, 2.99 mmoles) in methylene chloride (5 ml) was added. The mixture stirred at room temperature for ten minutes and calcium hydroxide (440 mg, 5.95 mmoles) was added. The mixture stirred at room temperature for thirty minutes. The mixture was filtered, the solid washed with methylene chloride and the filtrate evaporated to yield an oil. Silica gel chromatography of the oil afforded the title compound as a white foam.

NMR (ppm) ($CDCl_3$): 12.65 (1H, s), 8.0 (2H, d), 7.4-7.6 (3H, t), 6.35 (1H, d), 5.65 (1H, d), 4.1 (2H, t), 3.3 (2H, t), 2.7-3.1 (2H, m), 2.55 (3H, s), 2.1-2.7 (5H, m), 1.1-1.6 (2H, m), 0.9 (3H, t), 0.7 (3H, d).

EXAMPLE 77

(Beta S*, gamma R*) sodium 4-(3-(4-acetyl-3-hydroxy-2-propylphenoxy)propylsulfonyl)-gamma-hydroxy-beta-methylenzenebutanoate

The compound of Example 76 (543 mg, 1.15 mmoles) was dissolved in THF (17 ml) and 1N NaOH (1.7 ml, 1.72 mmoles) was added. The mixture stirred for twelve hours at room temperature and was then concentrated to dryness. The residue was purified on XAD-8 resin to afford the title compound as a beige foam.

Analysis, calculated: C, 58,35; H, 6.07; S, 6.23. Observed: C, 58.28; H, 6.08; S, 6.13.

## EXAMPLE 78

(Beta S*, gamma R*)-sodium 4-(3-(4-acetyl-3-hydroxy-2-propylphenoxy)propylthio)-gamma-hydroxy-beta-methylbenzenebutanoate

Following the procedure of Example 77, but substituting an equivalent amount of the compound of Example 75, Step G, for the compound of Example 76, there was obtained the title compound as a beige foam. Analysis, calculated: C, 62.22; H, 6.48; S, 6.64. Observed: C, 62.34; H, 6.23; S, 6.74.

## EXAMPLE 79

(Beta R*, gamma R*) 4-(3-(4-acetyl-3-hydroxy-2-propylphenoxy)propylthio)-gamma-hydroxy-beta-methylbenzenebutanoic acid gamma lactone

Following the procedure of Example 75, Step G, but substituting the *trans*-lactone of Step F for the *cis*-lactone of Step F, there was obtained the title compound as an oil.
NMR (ppm) ($CDCl_3$): 12.75 (1H, s), 7.6 (1H, d), 7.2-7.5 (4H, dd), 6.4 (1H, d), 4.9 (1H, d), 4.15 (2H, t), 3.15 (2H, t), 2.6 (3H, s), 2.0-3.0 (7H, m), 1.3-1.8 (2H, m), 1.2 (2H, d), 0.95 (3H, t).

## EXAMPLE 80

(Beta R*, gamma R*) 4-(3-(4-acetyl-3-hydroxy-2-propylphenoxy)propylsulfonyl)-gamma-hydroxy-beta-methylbenzenebutanoic acid gamma lactone

Following the procedure of Example 76, but substituting an equivalent amount of the compound of

Example 79, for the compound of Example 75, there was obtained the title compound as a white foam.
NMR (ppm) ($CDCl_3$): 12.75 (1H, s), 8.05 (2H, d), 7.6 (3H, d), 6.4 (1H, d), 5.05 (1H, d), 4.15 (2H, t), 3.4 (2H, t), 2.65 (3H, s), 2.1-2.9 (7H, m), 1.25-1.7 (2H, m), 1.25 (3H, d), 0.9 (3H, t).

EXAMPLE 81

Sodium (beta R*, gamma R*) 4-(3-(4-acetyl-3-hydroxy-2-propylphenoxy)propylsulfonyl)-gamma-hydroxy-beta-methylbenzenebutanoate

Following the procedure of Example 77, but substituting an equivalent amount of the compound of Example 80 for the compound of Example 76, there was obtained the title compound as a beige foam.
Analysis, calculated: C, 58.28; H, 6.08; S, 6.13.
Observed: C, 58.10; H, 6.07; S, 6.37.

EXAMPLE 82

Sodium (beta R*, gamma R*)-4-(3-(4-acetyl-3-hydroxy-2-propylphenoxy)propylthio)-gamma-hydroxy-beta-methylbenzenebutanoate

Following the procedure of Example 78, but substituting an equivalent amount of the compound of Example 79 for the compound of Example 75, Step G, there was obtained the title compound as a beige foam.
Analysis, calculated: C, 62.34; H, 6.23; S, 6.74.
Observed: C, 62.40; H, 6.30; S, 6.87.

EXAMPLE 83

(Alpha S*, beta R*, gamma R*)-4-(3-(4-acetyl-3-hydroxy-2-propylphenoxy)propylthio)-gamma-hydroxy-alpha, beta-dimethyl-benzenebutanoic acid gamma lactone

A: <u>(Alpha S*, beta R*) 4-(3-bromopropylthio)-gamma-oxo-alpha,beta-dimethyl-benzenebutanoic acid</u>

3-Bromopropylthiobenzene (4.62 g, 20.0 mmoles) and meso-2,3-dimethyl succinic anhydride (2.56 g, 20.0 mmoles) were dissolved in dichloroethane (100 ml) and cooled to 0°. Aluminum chloride (5.32 g, 40.0 mmoles) was added to the solution and the reaction mixture stirred at room temperature for twelve hours. The mixture was poured into ice-water (300 ml) and acidified with 6N HCl (30 ml). Methylene chloride (200 ml) was added and the mixture was stirred for two hours. The organic layer was collected. The aqueous layer was extracted with methylene chloride and the combined organics were washed with brine, dried ($Na_2SO_4$) and concentrated, to afford the title compound.
NMR (ppm) ($CDCl_3$): 1.23 (3H, d), 1.30 (3H, d), 2.23 (2H, m), 2.8-3.3 (3H, m), 3.4-3.8 (3H, m), 7.37 (2H, d), 7.93 (2H, d), 10.3 (1H, broad).

B: <u>(Alpha S*, beta R*, gamma R*)-4(3-bromopropylthio)-gamma-hydroxy-alpha, beta-dimethylbenzenebutanoic acid gamma lactone</u>

To a solution of the compound of Step A above (5.2 g, 14.7 mmoles) in dioxane (20 ml) and water (5 ml) at 0° was added in NaOH (15 ml). Sodium borohydride (0.6 g, 15.0 mmoles) was added and the mixture stirred at from temperature for one hour. Cesium chloride (20 mg) was added and the mixture stirred for ten hours. The reaction mixture was acidified with concentrated HCl and extracted with ether. The organic extracts were concentrated,

dissolved in methylene chloride (20 ml) and trifluoroacetic acid (2 drops) was added. The solution stirred at room temperature for thirty minutes, was washed with brine and dried ($Na_2SO_4$). The resulting lactones were separated by HPLC to yield (a) a less polar lactone the (alpha R*, beta R*, gamma R*) isomer of the title compound and (b) a more polar lactone. NMR analysis confirmed the more polar lactone product (b) as the title compound.
NMR (ppm) ($CDCl_3$): 0.53 (3H, d), 1.23 (3H, d), 2.17 (2H, m), 2.67-3.17 (4H, m), 3.53 (2H, t), 5.50 (1H, d, J=5Hz), 7.23 (2H, d), 7.40 (2H, d).

C: Preparation of sodium (alpha S*, beta R*, gamma R*)-4(3-bromopropylthio)-gamma-hydroxy-alpha, beta-dimethylbenzenebutanoate

The more polar lactone of Step B above (822 mg, 2.40 mmoles) was dissolved in THF (20 ml) and treated with 2N NaOH (1.3 ml, 2.6 mmoles) at room temperature for fifteen hours. The solvent was removed *in vacuo*. The residue was dissolved in methanol and again concentrated *in vacuo* to yield the title compound as a foam. This compound was used without further purification or characterization in the next step.

D: (Alpha S*, beta R*, gamma R*)-4-(3-(4-acetyl-3-hydroxy-2-propylphenoxy)propylthio)-gamma-hydroxy-alpha, beta-dimethylbenzenebutanoic acid gamma lactone

2,4-Dihydroxy-3-propylacetophenone (450 mg, 2.32 mmoles) the compound of Step C above (890 mg, 2.32 mmoles) and potassium carbonate (1.09 g, 7.24

mmoles) were ref] ›ed in methyl ethyl ketone (30 ml) for four hours. ..he mixture was cooled to room temperature and was poured into ice-water. The mixture was acidified with 6N HCl and extracted with methylene chloride. The combined extracts were washed with brine and concentrated *in vacuo*. The residue was dissolved in methylene chloride (20 ml) and trifluoroacetic acid (5 drops) was added. After five minutes the solution was washed with water, 0.1N NaOH and brine. The organic layer was dried ($Na_2SO_4$) and concentrated *in vacuo*. The residue was purified by chromatography on silica gel to afford the title compound.

NMR (ppm) ($CDCl_3$): 0.57 (3H, d), 0.93 (3H, t), 1.23 (3H, d), 1.37-1.67 (2H, m), 2.0-2.3 (2H, m), 2.57 (3H, s), 2.5-3.0 (4H, m), 3.13 (2H, t), 4.13 (2H, t), 5.45 (1H, d, *J*=5Hz), 6.37 (1H, d, J=9Hz), 7.17 (2H, d, J=8Hz), 7.33 (2H, d, J=8Hz), 7.53 (1H, d, J=9Hz), 12.70 (1H, s).

## EXAMPLE 84

Sodium (alpha S*, beta R*, gamma R*)-4(3-(4-acetyl-3-hydroxy-2-propylphenoxy)propylthio)-gamma-hydroxy-alpha, beta-dimethylbenzenebutanoate

The lactone of Example 83, Step D (487 mg, 1.067 mmoles) was dissolved in THF (10 ml) and methanol (2 ml). To this solution was added 1N NaOH (2 ml) and the reaction mixture stirred at room temperature for twenty one hours. The mixture was concentrated *in vacuc* and the residue purified by chromatography on XAD-8 resin to yield the title compound.

Analysis, calculated: C, 62.88; H, 6.70; S, 6.46.
Observed: C, 63.02; H, 6.86; S, 6.37.

EXAMPLE 85

D,L-4-(3-(4-acetyl-3-hydroxy-2-propylphenoxy)-propylthio)-gamma-hydroxybenzenebutanoic acid gamma lactone

A: Preparation of 4-mercapto-gamma-oxo-benzene-butanoic acid, methyl ester

Sodium (280 mg, 12.18 mmoles) was dissolved in anhydrous methanol (50 ml). To the resulting solution was added the compound of Example 75, Step D (5.0 g). The mixture was stirred at room temperature for twelve hours and poured into a mixture of water (30 ml) and concentrated HCl (7 ml). The resulting yellow solid was collected by filtration, washed with water and dried to afford the title compound, m.p. 83-84°.

B: Preparation of 4-((3-(4-Acetyl-3-hydroxy-2-propylphenoxy)propylthio)-gamma-oxobenzenebutanoic acid

2-Hydroxy-3-propyl-4-(3-bromopropyloxy)acetophenone (2.4 mmoles) and the compound of Step A above (0.6 g, 2.4 mmoles) were dissolved in THF (50 ml) and heated at reflux for forty-eight hours in the presence of 1.0 equivalent of potassium carbonate. The reaction mixture was filtered and concentrated *in vacuo*. The residue was purified by chromatography on silica gel and saponified with potassium hydroxide (1.5 equivalents) in a mixture of methanol and water (25 ml, 10:1). The reaction mixture was concentrated *in vacuo*, the residue dissolved in water and acidified with citric acid. The aqueous phase was extracted with ethyl acetate. The organic phase was washed with brine, dried ($Na_2SO_4$) and evaporated

to dryness. The residue was triturated with hexane and the solid collected by filtration to afford the title compound.

Analysis, calculated: C, 64.98; H, 6.14; S, 7.23.
Observed: C, 65.09; H, 6.09; S, 7.28.

C: Preparation of D,L-4-(3-(4-acetyl-3-hydroxy-2-propylphenoxy)propylthio)-gamma-hydroxybenzenebutanoic acid gamma lactone

The compound of Step B above (4.0 g, 8.99 mmoles) was dissolved in a mixture of THF and ethanol (110 ml, 10:1). To this solution was added sodium borohydride (440 mg, 11.63 mmoles). The reaction mixture stirred for twenty-four hours and was concentrated *in vacuo*. The residue was treated with chloroform (40 ml) and acidified with trifluoroacetic acid. After two hours the reaction mixture was concentrated *in vacuo* and the residue purified by HPLC to afford the title compound.

Analysis, calculated: C, 67.27; H, 6.59; S, 7.41.
Observed: C, 67.49; H, 6.90; S, 7.81.

## EXAMPLE 86

D,L-4-(3-(4-acetyl-3-hydroxy-2-propylphenoxy)-propylsulfonyl)-gamma-hydroxybenzenebutanoic acid gamma lactone

A: Preparation of 4-(3-(4-acetyl-3-hydroxy-2-propylphenoxy)propylsulfonyl)-gamma-oxo-benzenebutenoic acid

The ester prepared in Example 85 Step B, was dissolved in methylene chloride (50 ml) and treated at room temperature with m-chloroperbenzoic acid (2.5 equivalents) for two hours. The reaction mixture was

concentrated *in vacuo* and purified by chromatography on silica gel to yield the methyl ester of the title compound, which, upon saponification with NaOH in methanol, yielded the title compound.
Analysis, calculated: C, 60.48; H, 5.92; S, 6.72
Observed: C, 60.51; H, 5.90; S, 6.48

B: Preparation of D,L-4-(3-(4-acetyl-3-hydroxy-2-propylphenoxy)propylsulfonyl)-gamma-hydroxybenzenebutanoic acid gamma lactone

The compound of Step A above (5.0 g, 10.5 mmoles) was dissolved in a mixture of THF-ethanol (105 ml, 20:1). To this solution was added sodium borohydride (440 mg, 11.63 mmoles) and the reaction mixture stirred at room temperature for 16 hours. The mixture was concentrated *in vacuo* and dissolved in chloroform (100 ml) containing trifluoroacetic acid (2 ml). This solution stirred at room temperature for two hours and was concentrated *in vacuo*. The residue was purified by HPLC to afford the title compound, m.p. 131-133°.
Analysis, calculated: C, 62.59; H, 6.13; S, 6.96.
Observed: C, 62.62; H, 6.09; S, 6.74.

Using the above described methodology and starting with the appropriately substituted 2,4-dihydroxyacetophenone, the following compounds are also prepared:

## EXAMPLE 87

4-((3-(4-acetyl-6-fluoro-3-hydroxy-2-propylphenoxy)propyl)thio)-gamma-oxobenzenebutanoic acid

EXAMPLE 88

4-((3-(4-acetyl-6-chloro-3-hydroxy-2-propylphenoxy) propyl)thio)gamma-oxobenzenebutanoic acid

EXAMPLE 89

4-(3-(4-acetyl-6-chloro-3-hydroxy-2-propylphenoxy) propylsulfonyl)-gamma-oxobenzenebutanoic acid

EXAMPLE 90

4-((3-(4-acetyl-6-bromo-3-hydroxy-2-propylphenoxy) propyl)thio)-gamma-oxobenzenbutanoic acid

EXAMPLE 91

4-(3-(4-acetyl-6-bromo-3-hydroxy-propylphenoxy) propylsulfonyl)-gamma-oxobenzenebutanoic acid

EXAMPLE 92

4-((3-(4-acetyl-3-hydroxy-6-methyl-2-propylphenoxy) propyl)thio)-gamma-oxobenzenebutanoic acid

EXAMPLE 93

(Beta S*, gamma R*) 4-(3-(4-acetyl-6-fluoro-3-hydroxy-2-propylphenoxy)propylthio-gamma-hydroxy-beta-methylbenzenebutanoic acid

EXAMPLE 94

(Beta S*, gamma R*) 4-(3-(4-acetyl-5-chloro-3-hydroxy-2-propylphenoxy)propylthio)-gamma-hydroxy-beta-methylbenzenebutanoic acid

EXAMPLE 95

(Beta S*, gamma R*) 4-(3-(4-acetyl-3-hydroxy-6-methyl-2-propylphenoxy)propylthio)-gamma-hydroxy-beta-methylbenzenebutanoic acid

EXAMPLE 96

(Beta S*, gamma R*) 4-(3-(4-acetyl-6-ethyl-3-hydroxy-2-propylphenoxy)propylthio)-gamma-hydroxy-beta-methylbenzenebutanoic acid

EXAMPLE 97

4-(3-(4-acetyl-6-fluoro-3-hydroxy-2-propylphenoxy)propyloxy)-gamma-hydroxy-beta,beta-dimethylbenzenebutanoic acid

EXAMPLE 98

4-(3-(4-acetyl-6-chloro-3-hydroxy-2-propylphenoxy)propyloxy)-gamma-hydroxy-beta,beta-dimethylbenzenebutanoic acid

EXAMPLE 99

4-(3-(4-acetyl-3-hydroxy-6-methyl-2-propylphenoxy)propyloxy)-beta-hydroxy-beta,beta,dimethylbenzenebutanoic acid

EXAMPLE 100

4-(3-(4-acetyl-6-fluoro-3-hydroxy-2-propylphenoxy)propylthio)-gamma-hydroxy-beta,beta-dimethylbenzenebutanoic acid

EXAMPLE 101

4-(3-(4-acetyl-5-chloro-3-hydroxy-2-propylphenoxy)propylthio)-gamma-hydroxy-beta,beta-dimethylbenzenebutanoic acid

EXAMPLE 102

4-(3-(4-acetyl-3-hydroxy-6-methyl-2-propylphenoxy)propylthio)-gamma-hydroxy-beta,beta-dimethylbenzen-butanoic acid

EXAMPLE 103

Sodium (beta S*, gamma R*) 4-(3-(4-acetyl-3-hydroxy-2-propylphenoxy)propyloxy)-gamma-hydroxy-beta-methylbenzenebutanoate

A: Preparation of methyl 4-hydroxy-gamma-oxo-beta-methylbenzenebutanoate

A mixture of the ester from Example 70, Step A (8.15 g), 48% HBr (40 ml) and acetic acid (80 ml) was refluxed for 4 days, then poured into water (250 ml) and extracted with ethyl acetate. The extracts were washed with water and dried ($Na_2SO_4$) and evaporated to an oil which was treated with 10% HCl in methanol (150 ml) for 18 hours. The volatiles were removed by evaporation and the residue was taken up in $CH_2Cl_2$, washed with water, brine, dried, evaporated to dryness then purified by chromatography on silica gel to provide the title compound, mp 89-91°C.

B: Preparation of methyl 4(3-bromopropoxy)-gamma-oxo-beta-methylbenzenebutanoate

The mixture of the phenol from Step A (4.0 g) 1,3-dibromopropane (18.2 ml), potassium carbonate (7..5 g) and methyl ethyl ketone (100 ml) was refluxed for 1 hour. The mixture was filtered, concentrated *in vacuo* and the residue was purified by chromatography on silica gel to provide the title compound as an oil.

C: Preparation of (beta S*, gamma S*) and (beta S*, gamma R*) 4-(3-bromopropyloxy)-gamma-hydroxy-beta-methylbutanoic acid gamma lactones

The ketone from Step B (5.0 g) in methanol (70 ml) was treated with ceric chloride monohydrate

(50 mg) and the mixture was cooled to -20°C followed by addition of sodium borohydride (277 mg). After stirring 2 hours at -20°C the mixture was warmed to ambient temperature, diluted with water (150 ml) and 1N HCl (10 ml), then extracted with $CH_2Cl_2$. The extracts were washed with brine and evaporated to an oil which was treated in methanol (50 ml) and 2N NaOH (22 ml) for 1 hour. The mixture was diluted with water (750 ml), acidified, and extracted with EtOAc. The organic extracts were washed with brine, dried ($Na_2SO_4$) evaporated to an oil which was dissolved in dichloromethane (100 ml) and trifluoroacetic acid (1 ml). After 30 minutes the mixture was evaporated to dryness and the residue was purified by chromatography on silica gel to provide the (beta S*, gamma S*)-isomer of the title compound (less polar). NMR ($CDCl_3$): 1.15 (3H, d), 2.1-2.9 (5H, m), 3.55 (2H, t), 4.1 (2H, t), 4.85 (1H, d), 6.9 (2H, d), 7.25 (2H, d), and the (beta S*, gamma R*) isomer of the title compound (more polar), NMR ($CDCl_3$): 0.7 (3H, d), 2.2-2.9 (5H, m), 3.6 (2H, t), 4.1 (2H, 5), 5.5 (1H, d), 6.9 (2H, d), 7.1 (2H, d).

D: Preparation of (Beta S*, gamma R*) 4-(3-(4-acetyl-3-hydroxy-2-propylphenoxy)propyloxy)-gamma-hydroxy-beta-methylbenzenebutanoic acid gamma lactone

The more polar isomer from Step C (1.2 g) and 2,4-dihydroxy-3-propylacetophenone (1.19 g) was refluxed in methyl ethyl ketone (20 ml) and potassium carbonate (2 g) for 12 hours. The mixture was filtered, concentrated and purified by chromatography on silica gel to provide the title compound as an

oil; NMR ($CDCl_3$): 0.7 (3H, d), 0.9 (3H, t) 1.5 (2H, m), 2.1-2.9 (7H, m), 2.5 (3H, s), 4.05-[illegible].3 (4H, ), 5.5 (1H, d), 6.4 (1H, d), 6.85 (2H, d), 7.1 (2H, d), 7.55 (2H, d), 12.65 (1H, s).

E: Preparation of Sodium (Beta S*, gamma R*) 4-(3-(4-acetyl-3-hydroxy-2-propylphenoxy)propyloxy)-gamma-hydroxy-beta-methylbenzenebutanoate

The lactone from Step D (1.19 g) was saponified in 1N NaOH (4 ml), THF (15 ml) and methanol (2 ml) for 12 hours. The mixture was concentrated *in vacuo* and the residue in water was purified on XAD-8 resin to provide the title compound as a foam.
Analysis, calculated: C, 64.36; H, 6.70.
Observed: C, 64.26; H, 6.93.

Using the above described methodologies, the following compounds are also prepared:

## EXAMPLE 104

4-(3-(4-acetyl-3-hydroxy-2-propylphenoxy)propylthio)-3-propyl-gamma-oxo-benzenebutanoic acid, m.p. 148-149°.
Analysis, calculated: C, 66.64; H, 7.04; S, 6.59.
Observed: C, 66.89; H, 7.22; S, 6.25.

## EXAMPLE 105

4-(3-(4-acetyl-3-hydroxy-2-propylphenoxy)propylsulfonyl)-3-propyl-gamma-oxo-benzenebutanoic acid, m.p. 156-158°C.
Analysis, calculated: C, 62.53; H, 6.61; S, 6.18.
Observed: C, 62.35; H, 6.62; S, 5.99.

EXAMPLE 106

(Beta R*, gamma S*) sodium 4(3(4-acetyl-3-hydroxy-2-propylphenoxy)propylthio)-3-fluoro-gamma-hydroxy-beta-methylbenzenebutanoate

EXAMPLE 107

(Beta R*, gamma S*) sodium 4-(3-(4-acetyl-3-hydroxy-2-propylphenoxy)propylthio)-3-bromo-gamma-hydroxy-beta-methylbenzenebutanoate

EXAMPLE 108

(Beta R*, gamma S*) sodium 4-(3-(4-acetyl-3-hydroxy-2-propylphenoxy)propylthio)-3-chloro-gamma-hydroxy-beta-methylbenzenebutanoate

EXAMPLE 109

4-(3-(4-acetyl-3-hydroxy-2-propylphenoxy)propylsulfonyl)-gamma-oxo-beta,beta-dimethylbenzenebutanoic acid

EXAMPLE 110

D,L-Sodium 4-(3-(4-acetyl-3-hydroxy-2-propylphenoxy)-propylsulfonyl)-gamma-hydroxy-beta,beta-dimethyl benzenebutanoate

EXAMPLE 111

4-(3-(4-acetyl-3-hydroxy-2-propylphenoxy)propylthio)-3-propyl-gamma-oxo-beta,beta-dimethylbenzenebutanoic acid

EXAMPLE 112

D,L Sodium 4-(3-(4-acetyl-3-hydroxy-2-propylphenoxy) propylthio)-3-propyl-gamma-hydroxy-beta,beta-dimethyl-benzenebutanoate

EXAMPLE 113

Sodium 4-(3-(4-acetyl-3-hydroxy-2-propylphenoxy) propylthio)-3-fluoro-gamma-oxo-beta,beta-dimethyl benzenebutanoate

EXAMPLE 114

D,L Sodium 4(3-(4-acetyl-3-hydroxy-2-propylphenoxy) propylthio)-3-fluoro-gamma-hydroxy-beta,beta-dimethylbenzenebutanoate

EXAMPLE 115

4-(3-(4-acetyl-3-hydroxy-2-propylphenoxy)propylthio)-3-chloro-gamma-oxo-beta,beta-dimethylbenzenebutanoic acid

EXAMPLE 116

D,L.-Sodium 4-(3-(4-acetyl-3-hydroxy-2-propylphenoxy) propylthio)-3-chloro-gamma-hydroxy-beta,beta-dimethylbenzenebutanoate

EXAMPLE 117

D,L-Sodium 4-(3-(4-acetyl-3-hydroxy-2-propylphenoxy)-propylthio)-3-bromo-gamma-hydroxy-beta,beta-dimethylbenzenebutanoate

EXAMPLE 118

D,L-Sodium 4-(3-(4-acetyl-3-hydroxy-2-propylphenoxy)-propyloxy)-3-fluoro-gamma-hydroxy-beta,beta-dimethylbenzenebutanoate

EXAMPLE 119

D,L-Sodium 4-(3-(4-acetyl-3-hydroxy-2-propylphenoxy) propyloxy)-3-bromo-gamma-hydroxy-beta,beta-dimethylbenzenebutanoate

EXAMPLE 120

D,L-Sodium 4-(3-(4-acetyl-3-hydroxy-2-propylphenoxy) propyloxy)-3-chloro-gamma-hydroxy-beta,beta-dimethyl-benzenebutanoate

EXAMPLE 121

4-(3-(4-acetyl-3-hydroxy-2-propylphenoxy)propyloxy-3-fluoro-gamma-oxo-beta,beta-dimethylbenzenebutanoic acid

EXAMPLE 122

4-(3-(4-acetyl-2-hydroxy-2-propylphenoxy)propyloxy)-3-chloro-gamma-oxo-beta,beta-dimethylbenzenebutanoic acid

EXAMPLE 123

4-(3-(4-acetyl-3-hydroxy-2-propylphenoxy)propyloxy)-3-bromo-gamma-oxo-beta,beta-dimethylbenzenebutanoic acid

EXAMPLE 124

4-(3-(4-acetyl-3-hydroxy-2-propylphenoxy)propyloxy)-3-propyl-gamma-oxo-beta,beta-dimethylbenzenebutanoic acid

EXAMPLE 125

Sodium-(Beta R*, gamma S*) 3-(3-(4-acetyl-3-hydroxy-2-propylphenoxy)propylthio)-gamma-hydroxy-beta-methyl-benzenebutanoate

EXAMPLE 126

D,L-sodium 3-(3-(4-acetyl-3-hydroxy-2-propylphenoxy) propylthio)-gamma-hydroxy-beta,beta-dimethyl-benzenebutanoate

EXAMPLE 127

D,L-sodium 3-(3-(4-acetyl-3-hydroxy-2-propylphenoxy) propyloxy)-gamma-hydroxy-beta,beta-dimethylbenzene butanoate

EXAMPLE 128

3-(3-(4-acetyl-3-hydroxy-2-propylphenoxy)propylthio)-gamma-oxobenzenebutanoic acid

EXAMPLE 129

3-(3-(4-acetyl-3-hydroxy-2-propylphenoxy)propylthio)-gamma-oxo-beta,beta,dimethylbenzenebutanoic acid

EXAMPLE 130

3-(3-(4-acetyl-3-hydroxy-2-propylphenoxy)propyloxy)-gamma-oxo-beta,beta-dimethylbenzenebutanoic acid

EXAMPLE 131

(Beta R*, gamma S*) sodium 4-(3-(4-acetyl-3-hydroxy-2-propylphenoxy)propylthio)-gamma-hydroxy-beta-methylbenzene butanoate-S-oxide

EXAMPLE 132

(Beta R*, gamma S*) 4-(3-(4-acetyl-3-hydroxy-2-propylphenoxy)propylthio)-3-propyl-gamma-hydroxy-beta-methylbenzenebutanoic acid

EXAMPLE 133

4-(3-(4-acetyl-3-hydroxy-2-propylphenoxy)-propylsufonyl)-gamma-oxo-beta,beta-dimethylbenzenebutanoic acid

EXAMPLE 134

D,L-4 (3-(4-acetyl-3-hydroxy-2-propylphenoxy)-propylsulfonyl)-gamma-hydroxy-beta,beta-dimethylbenzenebutanoic acid

EXAMPLE 135

D,L-4-(3-(4-acetyl-3-hydroxy-2-propylphenoxy)-propylthio)-3-propyl-gamma-oxo-beta,beta-dimethylbenzenebutanoic acid

EXAMPLE 136

4-(3-(4-acetyl-3-hydroxy-2-propylphenoxy)-propylthio)-3-fluoro-gamma-oxo-3,3-beta,beta-dimethylbenzenebutanoic acid

EXAMPLE 137

D,L-4-[4-(3-(4-acetyl-3-hydroxy-2-propylphenoxy) propylthio)-3-fluoro-gamma-hydroxy-beta,beta-dimethylbenzenebutanoic aicd

EXAMPLE 138

(3R*, 4S*) 4-[4-[3-(4-acetyl-3-hydroxy-2-propylphenoxy)propylthio]-3-fluorophenyl]-3-methyl-gamma-butyrolactone

EXAMPLE 139

(3R*, 4S*) 4-[4-[3-(4-acetyl-3-hydroxy-2-propylphenoxy)propylthio]-3-chlorophenyl]-3-methyl-gamma-butyrolactone

EXAMPLE 140

(3 R*, 4 S*) 4-[4-[3-(4-acetyl-3-hydroxy-2-propylphenoxy)propylthio]-3-bromophenyl]-3-methyl-gamma-butyrolactone

EXAMPLE 141

(3 R*, 4 R*) 4-[4-[3-(4-acetyl-3-hydroxy-2-propylphenoxy)propylthio]-3-fluorophenyl]-3-methyl-gamma-butyrolactone

EXAMPLE 142

(3 R*, 4 R*) 4-[4-[3-(4-acetyl-3-hydroxy-2-propylphenoxy)propylthio]-3-bromophenyl]-3-methyl-gamma-butyrolactone

EXAMPLE 143

(3 R*, 4 R*) 4-[4-[3-(4-acetyl-3-hydroxy-2-propylphenoxy)propylthio]-3-chlorophenyl]-3-methyl-gamma-butyrolactone

EXAMPLE 144

(3 R*, 4 S*) 4-[4-[3-(4-acetyl-3-hydroxy-2-propylphenoxy)propyloxy]-phenyl]-3-methyl-butyrolactone

EXAMPLE 145

(Beta R*, gamma S*) 4-(3-(4-acetyl-3-hydroxy-2-propylphenoxy)propyloxy)-3-propyl-gamma-hydroxy-beta-methylbenzenebutanoic acid

EXAMPLE 146

4-(3-(4-acetyl-3-hydroxy-2-propylphenylthio)propyloxy)-3-fluoro-gamma-oxo-benzenebutanoic acid

m.p. 133-134°.

EXAMPLE 147

4-(3-(4-acetyl-3-hydroxy-2-propylphenylthio)propyloxy)-3-fluoro-gamma-oxo-benzenebutanoic acid S-oxide

m.p. 156-157°.

EXAMPLE 148

4-(3-(4-acetyl-3-hydroxy-2-propylphenylsulfonyl) propyloxy)-3-fluoro-gamma-oxo-benzenebutanoic acid

m.p. 182-184°.

EXAMPLE 149

4-(3-(4-acetyl-3-hydroxy-2-propylphenylthio) propylthio)-gamma-oxo-benzenebutanoic acid

m.p. 87-88.5°.

EXAMPLE 150

4-(3-(4-acetyl-3-hydroxy-2-propylphenylthio) propylsulfonyl)-gamma-oxo-benzenebutanoic acid

m.p. 135-138°.

It is understood that for those compounds which contain asymmetric centers, the present invention includes the racemic mixture as well as the individual resolved optical isomers.

EXAMPLE 151

TEST RESULTS

Following the procedure of assay B (Indomethacin-Induced Ulcer Assay), various compounds were tested for ulcer inhibition. For Table 151-1, % Inhibition was calculated as:

$$\frac{\text{Mean Ulcers (Control)} - \text{Mean Ulcers (Test)}}{\text{Mean Ulcers (Control)}} \times 100 = \%\ \text{Inh.}$$

TABLE 151-1

INDOMETHACIN-INDUCED ULCER ASSAY

| Test Compound | Dose (mg/kg) | % Inhib. |
|---|---|---|
| Example 78 | 0.03 | 14.1 |
| | 0.1 | 27.1 |
| | 0.3 | 50.1 |
| | 1 | 70.1 |
| | 3 | 74.1 |
| | 10 | 76.1 |
| Example 103 | 0.1 | 61.1 |
| | 1 | 76.1 |
| | 10 | 74.1 |
| Example 77 | 30 | 46.5 |
| Example 29 | 30 | 78.9 |
| Example 40 | 30 | 89.4 |

CLAIMS

1. Use of leukotriene antagonists to produce cytoprotective pharmaceutical compositions.

2. Use according to claim 1 characterized in that the leukotriene antagonist is a compound of the formulae XI or XII:

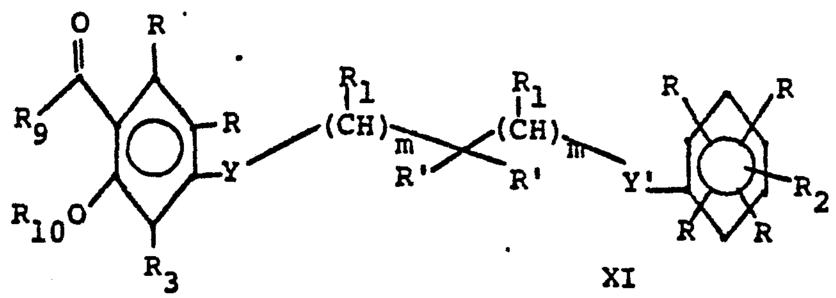

XI

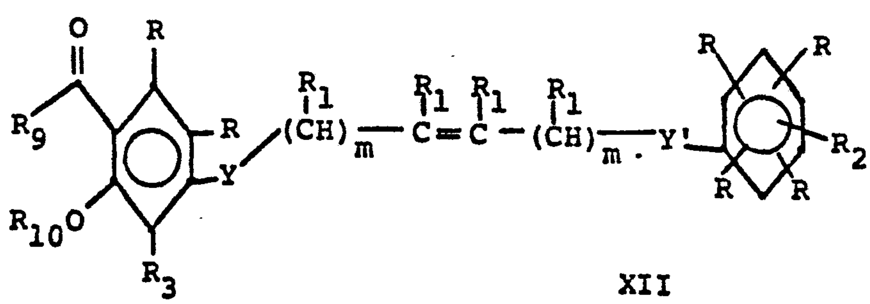

XII

wherein

each R is independently H, OH, alkyl of 1 to 6 carbon atoms which may be straight chain or branched; alkenyl of 2 to 6 carbon atoms which may be straight chain or branched; trifluoromethyl; alkoxy of 1 to 6 carbon atoms which may be straight chain or branched; SH; thioalkyl of 1 to 6 carbon atoms which may be straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R' is independently $R_4$; $OR_4$; $COOR_4$; $N(R_4)_2$; $SR_4$; $CH_2OR_4$; CHO; or together R' and R' are O; $CH_2$; or 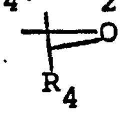 ;

Y is oxygen, sulfur, sulfoxide, sulfone; $\overset{O}{\overset{\|}{S}}=NR_{11}$ wherein $R_{11}$ is alkyl of 1-4 carbon atoms which may be straight chain or branched; $NR_{12}$ wherein $R_{12}$ is H, alkyl of 1-4 carbon atoms which may be straight chain or branched; or $N-\overset{O}{\overset{\|}{C}}-R_{13}$ wherein $R_{13}$ is alkyl of 1-4 carbon atoms which may be straight chain or branched, alkoxy of 1-4 carbon atoms which may be straight chain or branched; or N-CN;

Y' is Y, $-CH_2-$ or $-\overset{O}{\overset{\|}{C}}-$;

each $R_1$ is independently hydrogen or alkyl of 1-3 carbon atoms;

each m is independently an integer from 0-6; and

$R_2$ is $$-\overset{Z}{\overset{\|}{C}}-\left(\underset{R_7}{\overset{R_6}{C}}\right)_r-\left[\overset{R_8}{C}=\overset{R_8}{C}\right]_p-\left(\underset{R_7}{\overset{R_6}{C}}\right)_q-R_5$$

wherein Z is O, S, $CH_2$, H and OH, alkenyl of 1-4 carbons; or $N-R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of 1to 3 carbon atoms; halogen, hydroxy, haloalkyl, COOH, CN, formyl or acyl of 1 to 6 carbon atoms;

each $R_6$ is independently H or alkyl of 1-4 carbons;

each $R_7$ is independently H, OH, or alkyl of 1-4 carbons;

each $R_8$ is independently H, or alkyl of 1-4 carbons, and is absent when a triple bond is present;

$R_5$ is $COOR_4$; $CH_2OH$; CHO; tetrazole; $NHSO_2R_{14}$; $CONHSO_2R_{14}$ hydroxymethylketone; CN; $CON(R_7)_2$; a monocyclic or bicyclic heterocyclic ring containing an acidic hydroxyl group; or $COOR_{15}$ where $R_{15}$ is:

$-(-CH_2)_s-\overset{R_6}{\underset{R_6}{C}}-(CH_2)_s-R_{16}$ wherein each s is independently 0-3; $R_{16}$ is

A) a monocyclic or bicyclic heterocyclic radical containing from 3 to 12 nuclear carbon atoms and 1 or 2 nuclear heteroatoms selected from N and S with at least one being N, and with each ring in the heterocyclic radical being formed of 5 or 6 atoms, or

B) the radical $W-R_{17}$ wherein W is O, S or NH and $R_{17}$ contains up to 21 carbon atoms and is (1) a hydrocarbon radical or (2) an acyl radical of an organic acyclic or monocyclic

carboxylic acid containing not more than 1 heteroatom in the ring;

r an q are each independently 0-20 provided that the total of r and q does not exceed 20; and

p is 0 or 1;

$R_3$ is alkyl of 1 to 6 carbon atoms which may be straight chain or branched; or alkenyl of 3 to 6 carbon atoms which may be straight chain or branched as illustrated in Formulae IV and V;

$R_9$ is alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched; $R_4\overset{O}{\overset{\|}{C}}-$ or $R_4OCH_2-$;

and a pharmaceutically acceptable salt or acid addition salt thereof, or a compound of the Formula:

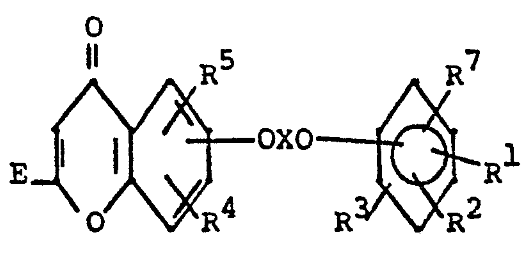

wherein

$R^1$, $R^2$, $R^3$, $R^4$, $R^5$ and $R^7$ may be the same or different, and are hydrogen, hydroxy, alkoxy, alkoxy substituted by phenyl, acyl, amino,

acylamino, alkenyl, halogen, or alkyl, provided that at least one of the $R^1$, $R^2$, $R^3$ and $R^7$ are other than hydrogen and hydroxy,

or an adjacent pair of $R^1$, $R^2$, $R^3$ and $R^7$ represent a chain —COCH═CH—O— ,

X is a hydrocarbon chain containing from 2 to 10 carbon atoms and optionally substituted by a hydroxy group, and

E is a carboxy group, a carboxyethyl group, or a tetrazole group, and pharmaceutically acceptable derivatives thereof, and pharmaceutically acceptable salts thereof.

3. Use according to patent claim 2 characterized in that the leukotriene antagonist is

(βS*, γR*)-sodium 4-(3-(4-acetyl-3-hydroxy-2-propylphenoxy)propylthio)-γ-hydroxy-β-methylbenzene butanoate,

(βS*, γR*) 4-(3-(4-acetyl-3-hydroxy-2-propylphenoxy) propyloxy)-γ-hydroxy-β-methyl-benzenebutanoic acid sodium salt,

(2,2-dimethyl-1-oxopropoxy)methyl 4-((3-(4-acetyl-3-hydroxy-2-propylphenoxy)propyl)sulfonyl)-γ-oxobenzenebutanoate,

(βS*, γR*)-sodium 4-((3-(4-acetyl-3-hydroxy-2-propylphenoxy)propyl)sulfonyl)-γ-hydroxy-β-methylbenzenebutanoate,

4-((3-(4-acetyl-3-hydroxy-2-propylphenoxy)propyl)-sulfonyl)-γ-oxobenzenebutanoic acid,

4-((3-(4-acetyl-3-hydroxy-2-propylphenoxy)propyl)thio-γ-oxobenzenebutanenitrile,

7-[3-(4-acetyl-3-hydroxy-2-propylphenoxy)-2-hydroxy-propoxy]-4-oxo-8-propyl-4H-1-benzopyran-2-carboxylic acid,
7-[3-(4-acetyl-3-hydroxy-2-propylphenoxy)-2-hydroxyl-propyloxy]-4-oxo-8-propyl-4H-1-benzopyran-2-proprionic acid, or
7-[3-(4-acetyl-3-hydroxy-2-propylphenoxy)propyloxy]-4-oxo-8-propyl-4H-1-benzopyran-2-proprionic acid.

4. Use according to one of the patent claims 1 - 3, characterized in that the leukotriene antagonist is used in such an amount to produce the cytoprotective pharmaceutical composition, that the produced composition can be administered in a dosage level from 0,001 mg to 100 mg of the leukotriene antagonists per kg body weight, preferably in a dosage level of 0,01 mg to 30 mg of the leukotriene antagonists per kg body weight.

5. Use according to one of the patent claims 1 - 4, characterized in that the leukotriene antagonist is used to produce a cytoprotective pharmaceutical composition having reduced undesirable side effects of an NSAID by incorporating into the pharmaceutical composition a cytoprotective-effective amount of the leukotriene antagonist and, furthermore, NSAID.

6. Use according to claim 5 characterized in that the weight ratio of leukotriene antagonist to NSAID in the produced pharmaceutical composition is

in the range from 200:1 to 1:200.

7. Process for the preparation of a cytoprotective pharmaceutical composition characterized in that into the pharmaceutical composition there is incorporated a cytoprotective effective amount of at least one leukotriene antagonist.

8. Process according to patent claim 7 characterized in that asleukotriene antagonist there is incorporated a compound of the formulae XI or XII:

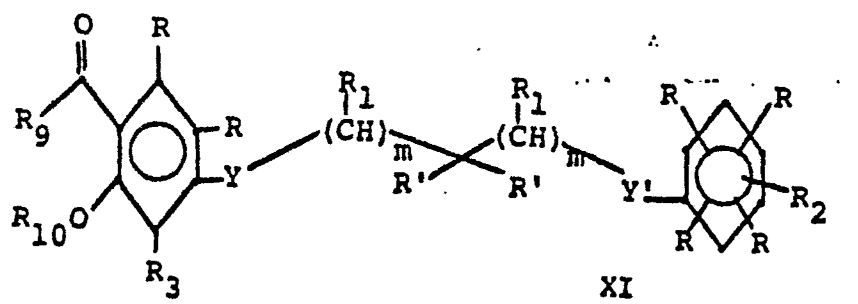

XI

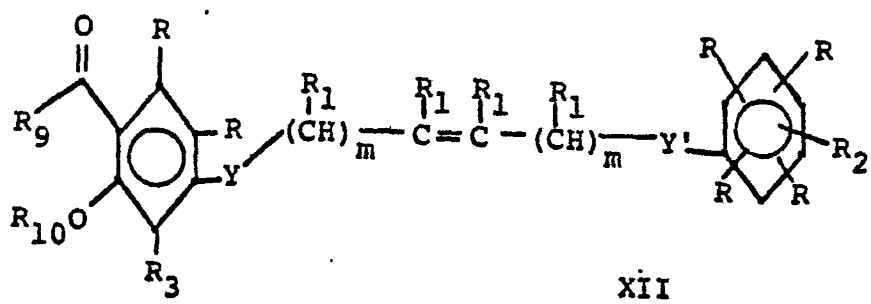

XII

wherein

each R is independently H, OH, alkyl of 1 to 6 carbon atoms which may be straight chain or branched; alkenyl of 2 to 6 carbon atoms which may be straight

chain or branched; trifluoromethyl; alkoxy of 1 to 6 carbon atoms which may be straight chain or branched; SH; thioalkyl of 1 to 6 carbon atoms which may be straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R' is independently $R_4$; $OR_4$; $COOR_4$; $N(R_4)_2$; $SR_4$; $CH_2OR_4$; CHO; or together R' and R' are O, $CH_2$; or 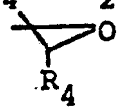 ;

Y is oxygen, sulfur, sulfoxide, sulfone; 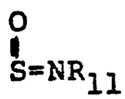 wherein $R_{11}$ is alkyl of 1-4 carbon atoms which may be straight chain or branched; $NR_{12}$ wherein $R_{12}$ is H, alkyl of 1-4 carbon atoms which may be straight chain or branched; or 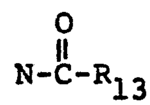 wherein $R_{13}$ is alkyl of 1-4 carbon atoms which may be straight chain or branched, alkoxy of 1-4 carbon atoms which may be straight chain or branched; or N-CN;

Y' is Y, $-CH_2-$ or 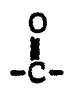;

each $R_1$ is independently hydrogen or alkyl of 1-3 carbon atoms;

each m is independently an integer from 0-6; and

$R_2$ is 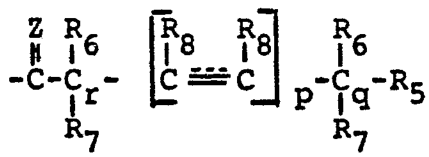

wherein Z is O, S, $CH_2$, H and OH, alkenyl of 1-4 carbons; or $N-R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of lto 3 carbon atoms, halogen, hydroxy, haloalkyl, COOH, CN, formyl or acyl of 1 to 6 carbon atoms;

each $R_6$ is independently H or alkyl of 1-4 carbons;

each $R_7$ is independently H, OH, or alkyl of 1-4 carbons;

each $R_8$ is independently H, or alkyl of 1-4 carbons, and is absent when a triple bond is present;

$R_5$ is $COOR_4$; $CH_2OH$; CHO; tetrazole; $NHSO_2R_{14}$; $CONHSO_2R_{14}$ hydroxymethylketone; CN; $CON(R_7)_2$; a monocyclic or bicyclic heterocyclic ring containing an acidic hydroxyl group; or $COOR_{15}$ where $R_{15}$ is:

$$-(-CH_2)_s-\underset{R_6}{\overset{R_6}{\underset{|}{\overset{|}{C}}}}-(CH_2)_s-R_{16}$$ wherein each s is independently 0-3; $R_{16}$ is

A) a monocyclic or bicyclic heterocyclic radical containing from 3 to 12 nuclear carbon atoms and 1 or 2 nuclear heteroatoms selected from N and S with at least one being N, and with each ring in the heterocyclic radical being formed of 5 or 6 atoms, or

B) the radical $W-R_{17}$ wherein W is O, S or NH and $R_{17}$ contains up to 21 carbon atoms and is (1) a hydrocarbon radical or (2) an acyl radical of an organic acyclic or monocyclic carboxylic acid containing not more than 1 heteroatom in the ring;

r an q are each independently 0-20 provided that the total of r and q does not exceed 20; and

p is 0 or 1;

$R_3$ is alkyl of 1 to 6 carbon atoms which may be straight chain or branched; or alkenyl of 3 to 6 carbon atoms which may be straight chain or branched as illustrated in Formulae IV and V;

$R_9$ i alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched; $R_4\overset{O}{\overset{\|}{C}}-$ or $R_4OCH_2-$;

or a pharmaceutically acceptable salt or acid addition salt thereof, or a compound of the Formula XIII

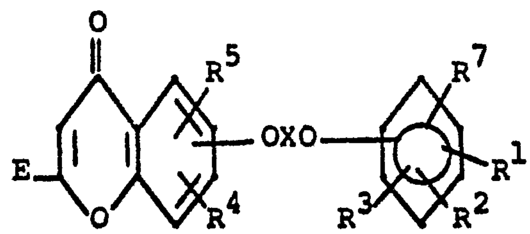

XIII

wherein

$R^1$, $R^2$, $R^3$, $R^4$, $R^5$ and $R^7$ may be the same or different, and are hydrogen, hydroxy, alkoxy, alkoxy substituted by phenyl, acyl, amino, acylamino, alkenyl, halogen, or alkyl, provided that at least one of the $R^1$, $R^2$, $R^3$ and $R^7$ are other than hydrogen and hydroxy,

or an adjacent pair of $R^1$, $R^2$, $R^3$ and $R^7$ represent a chain $-COCH{=}CH-O-$ ,

X is a hydrocarbon chain containing from 2 to 10 carbon atoms and optionally substituted by a hydroxy group, and

E is a carboxy group, a carboxyethyl group, or a tetrazole group or a pharmaceutically acceptable derivative thereof or a pharmaceutically acceptable salt thereof and that optionally a pharmaceutically acceptable carrier is incorporated into the cytoprotective pharmaceutical composition.

9. Process according to claim 8, characterized in that the leukotriene antagonist is

(ß*, γR*)-sodium 4-(3-(4-acetyl-3-hydroxy-2-propylphenoxy)propylthio)-γ-hydroxy-ß-methylbenzene butanoate,

(ßS*, γR*) 4-(3-(4-acetyl-3-hydroxy-2-propylphenoxy) propyloxy)-γ-hydroxy-ß-methylbenzenebutanoic acid sodium salt,

(2,2-dimethyl-1-oxopropoxy)methyl 4-((3-(4-acetyl-3-hydroxy-2-propylphenoxy)propyl)sulfonyl)-γ-oxobenzenebutanoate,

(ßS*, γR*)-sodium 4-((3-(4-acetyl-3-hydroxy-2-propylphenoxy)propyl)sulfonyl)-γ-hydroxy-ß-methylbenzenebutanoate,

4-((3-(4-acetyl-3-hydroxy-2-propylphenoxy)propyl)-sulfonyl)-γ-oxobenzenebutanoic acid,

4-((3-(4-acetyl-3-hydroxy-2-propylphenoxy)propyl)thio-γ-oxobenzenebutanenitrile,

7-[3-(4-acetyl-3-hydroxy-2-propylphenoxy)-2-hydroxypropoxy]-4-oxo-8-propyl-4H-1-benzopyran-2-carboxylic acid monosodium salt,

7-[3-(4-acetyl-3-hydroxy-2-propylphenoxy)-2-hydroxylpropyloxy]-4-oxo-8-propyl-4H-1-benzopyran-2-proprionic acid, or

7-[3-(4-acetyl-3-hydroxy-2-propylphenoxy)propyloxy]-4-oxo-8-propyl-4H-1-benzopyran-2-proprionic acid.

10. Process according to one of the patent claims 7 - 9, characterized in that a cytoprotective pharmaceutical composition is prepared which has less undesirable side effects, by incorporating into the pharmaceutical composition a further pharmaceutically active component, preferably a inhibitor of the biosynthesis of the leukotrienes, an antihistaminic agent, a prostaglandin antagonist, a thromboxane antagonist, a histidine decarboxylase inhibitor, a $H_1$ or $H_2$-receptor antagonist, a $K^+/H^+$ ATPase inhibitor and/or a NSAID.